(19) Europäisches Patentamt
European Patent Office
Office européen des brevets

(11) EP 2 480 084 B1

(12) **EUROPEAN PATENT SPECIFICATION**

(45) Date of publication and mention of the grant of the patent:
**12.11.2014 Bulletin 2014/46**

(21) Application number: **10819435.8**

(22) Date of filing: **23.09.2010**

(51) Int Cl.:
***A61K 31/4155*** (2006.01) ***A61K 31/519*** (2006.01)
***A61K 45/06*** (2006.01) ***A61P 35/00*** (2006.01)

(86) International application number:
**PCT/US2010/049946**

(87) International publication number:
**WO 2011/038082 (31.03.2011 Gazette 2011/13)**

(54) **PHARMACEUTICAL COMBINATION**

PHARMAZEUTISCHE KOMBINATION

COMBINAISON PHARMACEUTIQUE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **23.09.2009 US 245019 P**

(43) Date of publication of application:
**01.08.2012 Bulletin 2012/31**

(73) Proprietor: **GlaxoSmithKline LLC**
**Wilmington, Delaware 19808 (US)**

(72) Inventors:
- **DUMBLE, Melissa**
**Piscataway**
**NJ 08854 (US)**
- **GILMER, Tona**
**Research Triangle Park**
**NC 27709 (US)**
- **KUMAR, Rakesh**
**Collegeville**
**PA 19426 (US)**
- **LEBOWITZ, Peter, F.**
**Collegeville**
**PA 19426 (US)**
- **MORRIS, Shannon, Renae**
**Research Triangle Park**
**NC 27709 (US)**
- **LAQUERRE, Sylvie**
**King of Prussia**
**PA 19406 (US)**

(74) Representative: **Reed, Michael Antony et al**
**GlaxoSmithKline**
**Global Patents (CN925.1)**
**980 Great West Road**
**Brentford, Middlesex TW8 9GS (GB)**

(56) References cited:
**WO-A1-2008/098104** **WO-A1-2008/098104**
**US-A1- 2006 014 768** **US-A1- 2006 014 768**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 480 084 B1

## Description

### FIELD OF THE INVENTION

The present invention relates to a combination for use in the treatment of cancer in a mammal. In particular, the invention relates to a novel combination comprising the MEK inhibitor: N-{3-[3-cyclopropyl-5-(2-fluoro-4-iodo-phenylamino)6,8-dimethyl-2,4,7-trioxo-3,4,6,7-tetrahydro-2H-pyrido[4,3-d]pyrimidin-1-yl]phenyl}acetamide, or a pharmaceutically acceptable salt or solvate thereof, and the Akt inhibitor: *N*-{(1*S*)-2-amino-1-[(3,4-difluorophenyl)methyl]ethyl}-5-chloro-4-(4-chloro-1-methyl-1*H*-pyrazol-5-yl)-2-furancarboxamide, or a pharmaceutically acceptable salt thereof, pharmaceutical compositions comprising the same, and such combinations for use in the treatment of cancer.

### BACKGROUND OF THE INVENTION

Effective treatment of hyperproliferative disorders including cancer is a continuing goal in the oncology field. Generally, cancer results from the deregulation of the normal processes that control cell division, differentiation and apoptotic cell death. Apoptosis (programmed cell death) plays essential roles in embryonic development and pathogenesis of various diseases, such as degenerative neuronal diseases, cardiovascular diseases and cancer. One of the most commonly studied pathways, which involves kinase regulation of apoptosis, is cellular signaling from growth factor receptors at the cell surface to the nucleus (Crews and Erikson, Cell, 74:215-17, 1993).

Receptor tyrosine kinases (RTKs) activated by extracellular growth factors recruit intracellular proteins to the cell membrane, thereby activating key signal-transduction components that are commonly hyper-activated in cancers. These include phosphoinositide 3-kinase (hereinafter referred to as PI3K) and the guanosine triphosphate (GTP)-binding protein RAS.

The PI3K protein family consists of 15 members that share sequence homology, particularly within their kinase domains; however; they have distinct substrate specificities and modes of regulation (Vivanco & Sawyers. Nat. Rev. Cancer, 2002.2:489-501). Class I PI3-kinases phosphorylate inositol-containing lipids, known as phosphatidylinositols (PtdIns) at the 3 position. The primary substrate of Class I family members, PtdIns-4, 5-P2 (PIP2) is converted to PtdIns-3, 4, 5-P3 (PIP3) by these kinases. PIP3 is a critical second messenger which recruits proteins that contain pleckstrin homology domains to the cell membrane where they are activated. The most studied of these proteins is AKT which promotes cell survival, growth, and proliferation. AKT signaling can be regulated by PI3K activation. The three AKT gene family members, AKT1, AKT2 and AKT3 encode for serine/theonine-specific protein kinases, which, upon activation, moves to the cytoplasm and nucleus where it phosphorylates numerous substrates, including mTOR (TORC1).

The PI3K-AKT pathway is among the most commonly activated pathways in human cancer. The function and importance of this pathway in tumorigenesis and tumor progression is well established (Samuels & Ericson. Curr. Opp in Oncology, 2006. 18: 77-82). Thus, the deregulation of PI3K/AKT signaling in tumors contributes to a cellular phenotype that demonstrates numerous hallmarks of malignancies, which includes unlimited reproductive potential and the evasion of apoptosis (Hanahan & Weinberg, Cell. 2000. 100:57-70). Numerous germline and somatic genetic alterations can activate these pathways. Somatic activation of the PI3K/AKT signaling pathway most commonly occurs either through activating mutations in PI3KCA (which encodes the catalytic p110$\alpha$ kinase subunit) or through loss-of-function mutations, deletions or promoter methylation silencing of the tumor suppressor gene PTEN (a negative regulator of PI3K) (Vivanco & Sawyers. Nat. Rev. Cancer. 2002. 2:489-501). More rarely, an activating mutation of AKT1 leading to PI3K independent membrane recruitment has also been identified in breast, ovarian, and colorectal cancer (Carpten et al. Nature. 2007. 448:439-44).

Mitogen-activated protein (MAP) Kinase/extracellular signal-regulated kinase (ERK) kinase (hereinafter referred to as MEK) is known to be involved in the regulation of numerous cellular processes. The Raf family (B-Raf, C-Raf etc.) activates the MEK family (MEK-1, MEK-2 etc.) and the MEK family activates the ERK family (ERK-1 and ERK-2). Broadly, the signaling activity of the RAF/MEK/ERK pathway controls mRNA translation. This includes genes related to the cell cycle. Hence, hyperactivation of this pathway can lead to uncontrolled cell proliferation. Deregulation of the RAF/MEK/ERK pathway by ERK hyperactivation is seen in approximately 30% of all human malignancies (Allen, LF, et al. Semin. Oncol. 2003. 30(5 Suppl 16):105-16). RAS, which can signal through both the PI3K/AKT and RAF/MEK/ERK, has a mutated oncogenic protein in 15% of all cancers (Davies, H. et al. Nature. 2002. 417:949-54). Also, activating BRAF mutations have been identified at a high frequency in specific tumor types (e.g., melanomas) (Davies, H. et al. Nature. 2002. 417:949-54). Although activating mutations in MEK itself don't appear to frequently occur in human cancers, MEK is thought to be an important drug target for treating human cancer because of its central role in the ERK pathway. Further, MEK inhibitory activity effectively induces inhibition of ERK1/2 activity and suppression of cell proliferation (The Journal of Biological Chemistry, vol. 276, No. 4, pp. 2686-2692, 2001), and the compound is expected to show effects on diseases caused by undesirable cell proliferation, such as tumor genesis and/or cancer.

These observations demonstrate that the PI3K/Akt pathway plays important roles for regulating cell survival or

apoptosis in tumorigenesis and/or cancer.

These observations demonstrate that the RAF/MEK/ERK pathway plays important roles for regulating cell survival or apoptosis in tumorigenesis and/or cancer.

It would be useful to provide a novel therapy which provides more effective and/or enhanced treatment of an individual suffering the effects of cancer,

SUMMARY OF THE INVENTION

One embodiment of this invention provides a composition comprising a combination of :

(i) a compound of Structure (I):

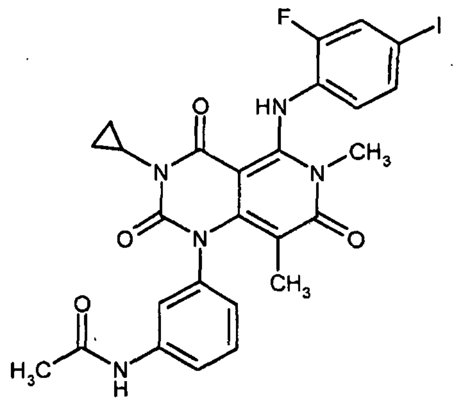

(I)

or a pharmaceutically acceptable salt or solvate thereof; and

(ii) a compound of Structure (II):

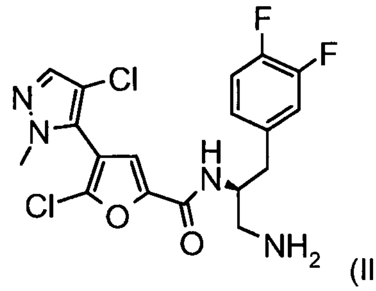

(II)

or a pharmaceutically acceptable salt thereof.

One embodiment of this invention provides a combination for use in treating cancer in a human in need thereof comprising a therapeutically effective amount of a combination of N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide, or a pharmaceutically acceptable salt or solvate, suitably the dimethyl sulfoxide solvate, thereof, and *N*-{(1*S*)-2-amino-1-[(3,4-difluorophenyl)methyl]ethyl}-5-chloro-4-(4-chloro-1-methyl-1*H*-pyrazol-5-yl)-2-furancarboxamide, or a pharmaceutically acceptable salt thereof.

One embodiment of this invention provides a combination for use in treating cancer in a human in need thereof comprising a therapeutically effective amount of a combination of N-{3-[3-cyclopropyl-5-(2-fluoro-4-iodo-phenylamino)6,8-dimethyl-2,4,7-trioxo-3,4,6,7-tetrahydro-2H-pyrido[4,3-d]pyrimidin-1-yl]phenyl}acetamide, or a pharmaceutically acceptable salt or solvate, suitably the dimethyl sulfoxide solvate, thereof, and *N*-{(1*S*)-2-amino-1-[(3,4-difluorophenyl)methyl]ethyl}-5-chloro-4-(4-chloro-1-methyl-1*H*-pyrazol-5-yl)-2-furancarboxamide, or a pharmaceutically acceptable salt thereof,

wherein the combination is administered within a specified period, and

wherein the combination is administered for a duration of time.

One embodiment of this invention provides a combination for use in treating cancer in a human in need thereof

comprising a therapeutically effective amount of a combination of N-{3-[3-cyclopropyl-5-(2-fluoro-4-iodo-phenylamino)6,8-dimethyl-2,4,7-trioxo-3,4,6,7-tetrahydro-2H-pyrido[4,3-d]pyrimidin-1-yl]phenyl}acetamide, or a pharmaceutically acceptable salt or solvate, suitably the dimethyl sulfoxide solvate, thereof, and *N*-{(1*S*)-2-amino-1-[(3,4-difluorophenyl)methyl]ethyl}-5-chloro-4-(4-chloro-1-methyl-1*H*-pyrazol-5-yl)-2-furancarboxamide, or a pharmaceutically acceptable salt thereof,
wherein the compounds of the combination are administered sequentially.

BRIEF DESCRIPTION OF THE DRAWINGS

Figure - 1 Figure 1 depicts the cell growth inhibition-dose response curves for MDA-MB-175-VII, BT474-J4 and JIMT-1.

Figure - 2 Figure 2 depicts the effect of combinations of Compounds A and B and monotherapy against KRAS mutant tumor xenografts growing in SCID mice.

DETAILED DESCRIPTION OF THE INVENTION

The present invention relates to combinations that exhibit antiproliferative activity. Suitably, the invention relates to a combination for use in treating cancer comprising N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide, or a pharmaceutically acceptable salt or solvate, suitably the dimethyl sulfoxide solvate, thereof, (hereinafter Compound A, or a pharmaceutically acceptable salt or solvate, suitably the dimethyl sulfoxide solvate, thereof,
which compound is represented by Structure I:

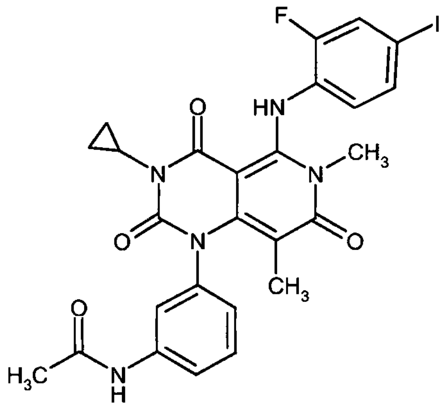

(I));

and N-{(1S)-2-amino-1-[(3,4-difluorophenyl)methyl]ethyl}-5-chloro-4-(4-chloro-1-methyl-1H-pyrazol-5-yl)-2-furancarboxamide or a pharmaceutically acceptable salt thereof, (hereinafter Compound B or a pharmaceutically acceptable salt thereof,
which compound is represented by Structure II:

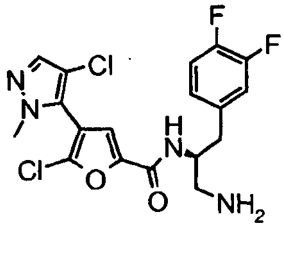

(II)),

for use in treating cancer.
Compound A is disclosed and claimed, along with pharmaceutically acceptable salts and solvates thereof, as being useful as an inhibitor of MEK activity, particularly in treatment of cancer, in International Application No.

PCT/JP2005/011082, having an International filing date of June 10, 2005; International Publication Number WO 2005/121142 and an International Publication date of December 22, 2005, the entire disclosure of which is hereby incorporated by reference, Compound A is the compound of Example 4-1. Compound A can be prepared as described in International Application No. PCT/JP2005/011082. Compound A can be prepared as described in United States Patent Publication No. US 2006/0014768, Published January 19, 2006, the entire disclosure of which is hereby incorporated by reference.

Suitably, Compound A is in the form of a dimethyl sulfoxide solvate. Suitably, Compound A is in the form of a sodium salt. Suitably, Compound A is in the form of a solvate selected from: hydrate, acetic acid, ethanol, nitromethane, chlorobenzene, 1-pentanci, isopropyl alcohol, ethylene glycol and 3-methyl-1-butanol. These solvates and salt forms can be prepared by one of skill in the art from the description in International Application No. PCT/JP2005/011082 or United States Patent Publication No. US 2006/0014768.

Compound B is disclosed and claimed, along with pharmaceutically acceptable salts thereof, as being useful as an inhibitor of AKT activity, particularly in treatment of cancer, in International Application No. PCT/US2008/053269, having an International filing date of February 7, 2008; International Publication Number WO 2008/098104 and an International Publication date of August 14, 2008, the entire disclosure of which is hereby incorporated by reference, Compound B is the compound of example 224. Compound B can be prepared as described in International Application No. PCT/US2008/053269.

The administration of a therapeutically effective amount of the combinations of the invention are advantageous over the individual component compounds in that the combinations will provide one or more of the following improved properties when compared to the individual administration of a therapeutically effective amount of a component compound: i) a greater anticancer effect than the most active single agent, ii) synergistic or highly synergistic anticancer activity, iii) a dosing protocol that provides enhanced anticancer activity with reduced side effect profile, iv) a reduction in the toxic effect proflie, v) an increase in the therapeutic window, or vi) an increase in the bioavailability of one or both of the component compounds.

The compounds of the invention may contain one or more chiral atoms, or may otherwise be capable of existing as two enantiomers. Accordingly, the compounds of this invention include mixtures of enantiomers as well as purified enantiomers or enantiomerically enriched mixtures. Also, it is understood that all tautomers and mixtures of tautomers are included within the scope of Compound A, and pharmaceutically acceptable salts or solvates thereof, and Compound B, and pharmaceutically acceptable salts thereof.

The compounds of the invention may form a solvate which is understood to be a complex of variable stoichiometry formed by a solute (in this invention, Compound A or a salt thereof and/or Compound B or a salt thereof) and a solvent. Such solvents for the purpose of the invention may not interfere with the biological activity of the solute. Examples of suitable solvents include water, methanol, dimethyl sulfoxide, ethanol and acetic acid. Suitably the solvent used is a pharmaceutically acceptable solvent. Examples of suitable pharmaceutically acceptable solvents include water, dimethyl sulfoxide, ethanol and acetic acid. Suitably the solvent used is water.

The pharmaceutically acceptable salts of the compounds of the invention are readily prepared by those of skill in the art.

When referring to a dosing protocol, the term "day", "per day" and the like, refer to a time within one calendar day which begins at midnight and ends at the following midnight.

By the term "treating" and derivatives thereof as used herein, is meant therapeutic therapy. In reference to a particular condition, treating means: (1) to ameliorate or prevent the condition of one or more of the biological manifestations of the condition, (2) to interfere with (a) one or more points in the biological cascade that leads to or is responsible for the condition or (b) one or more of the biological manifestations of the condition, (3) to alleviate one or more of the symptoms, effects or side effects associated with the condition or treatment thereof, or (4) to slow the progression of the condition or one or more of the biological manifestations of the condition. Prophylactic therapy is also contemplated thereby. The skilled artisan will appreciate that "prevention" is not an absolute term. In medicine, "prevention" is understood to refer to the prophylactic administration of a drug to substantially diminish the likelihood or severity of a condition or biological manifestation thereof, or to delay the onset of such condition or biological manifestation thereof. Prophylactic therapy is appropriate, for example, when a subject is considered at high risk for developing cancer, such as when a subject has a strong family history of cancer or when a subject has been exposed to a carcinogen.

As used herein, the term "effective amount" means that amount of a drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

By the term "combination" and derivatives thereof, as used herein is meant a therapeutically effective amount of Compound A, or a pharmaceutically acceptable salt or solvate thereof, and Compound B or a pharmaceutically

acceptable salt thereof for use in either simultaneous administration or any manner of separate sequential administration. Preferably, if the administration is not simultaneous, the compounds are administered in a close time proximity to each other. Furthermore, it does not matter if the compounds are administered in the same dosage form, e.g. one compound may be administered topically and the other compound may be administered orally. Suitably, both compounds are administered orally.

By the term "combination kit" as used herein is meant the pharmaceutical composition or compositions that are used to administer Compound A, or a pharmaceutically acceptable salt or solvate thereof, and Compound B, or a pharmaceutically acceptable salt thereof, according to the invention. When both compounds are administered simultaneously, the combination kit can contain Compound A, or a pharmaceutically acceptable salt or solvate thereof, and Compound B, or a pharmaceutically acceptable salt thereof, in a single pharmaceutical composition, such as a tablet, or in separate pharmaceutical compositions. When the compounds are not administered simultaneously, the combination kit will contain Compound A, or a pharmaceutically acceptable salt or solvate thereof, and Compound B, or a pharmaceutically acceptable salt thereof, in separate pharmaceutical compositions. The combination kit can comprise Compound A, or a pharmaceutically acceptable salt or solvate thereof, and Compound B, or a pharmaceutically acceptable salt thereof, in separate pharmaceutical compositions in a single package or in separate pharmaceutical compositions in separate packages.

In one aspect there is provided a combination kit comprising the components:

Compound A, or a pharmaceutically acceptable salt or solvate thereof, in association with a pharmaceutically acceptable carrier; and
Compound B, or a pharmaceutically acceptable salt thereof, in association with a pharmaceutically acceptable carrier.

In one embodiment of the invention the combination kit comprises the following components:

Compound A, or a pharmaceutically acceptable salt or solvate thereof, in association with a pharmaceutically acceptable carrier; and
Compound B, or a pharmaceutically acceptable salt thereof, in association with a pharmaceutically acceptable carrier, wherein the components are provided in a form which is suitable for sequential, separate and/or simultaneous administration.

In one embodiment the combination kit comprises:

a first container comprising Compound A, or a pharmaceutically acceptable salt or solvate thereof, in association with a pharmaceutically acceptable carrier; and
a second container comprising Compound B, or a pharmaceutically acceptable salt thereof, in association with a pharmaceutically acceptable carrier, and a container means for containing said first and second containers.

The "combination kit" can also be provided by instruction, such as dosage and administration instructions. Such dosage and administration instructions can be of the kind that is provided to a doctor, for example by a drug product label, or they can be of the kind that is provided by a doctor, such as instructions to a patient.

Unless otherwise defined, in all dosing protocols described herein, the regimen of compounds administered does not have to commence with the start of treatment and terminate with the end of treatment, it is only required that the number of consecutive days in which both compounds are administered and the optional number of consecutive days in which only one of the component compounds is administered, or the indicated dosing protocol - including the amount of compound administered, occur at some point during the course of treatment.

As used herein the term "Compound $A^2$" means --Compound A, or a pharmaceutically acceptable salt or solvate thereof---.

As used herein the term "Compound $B^2$" means --Compound B, or a pharmaceutically acceptable salt thereof---.

The term "loading dose" as used herein will be understood to mean a single dose or short duration regimen of Compound A or Compound B having a dosage higher than the maintenance dose administered to the subject to rapidly increase the blood concentration level of the drug. Suitably, a short duration regimen for use herein will be from: 1 to 14 days; suitably from 1 to 7 days; suitably from 1 to 3 days; suitably for three days; suitably for two days; suitably for one day. In some embodiments, the "loading dose" can increase the blood concentration of the drug to a therapeutically effective level. In some embodiments, the "loading dose" can increase the blood concentration of the drug to a therapeutically effective level in conjunction with a maintenance dose of the drug. The "loading dose" can be administered once per day, or more than once per day (e.g., up to 4 times per day). Suitably the "loading dose" will be administered once a day. Suitably, the loading dose will be an amount from 2 to 100 times the maintenance dose; suitably from 2 to 10 times; suitably from 2 to 5 times; suitably 2 times; suitably 3 times; suitably 4 times; suitably 5 times. Suitably, the

loading dose will be administered for from 1 to 7 days; suitably from 1 to 5 days; suitably from 1 to 3 days; suitably for 1 day; suitably for 2 days; suitably for 3 days, followed by a maintenance dosing protocol.

The term "maintenance dose" as used herein will be understood to mean a dose that is serially administered (for example., at least twice), and which is intended to either slowly raise blood concentration levels of the compound to a therapeutically effective level, or to maintain such a therapeutically effective level. The maintenance dose is generally administered once per day and the daily dose of the maintenance dose is lower than the total daily dose of the loading dose.

Suitably the combinations of this invention are administered within a "specified period".

By the term "specified period" and derivatives thereof, as used herein is meant the interval of time between the administration of one of Compound $A^2$ and Compound $B^2$ and the other of Compound $A^2$ and Compound $B^2$. Unless otherwise defined, the specified period can include simultaneous administration. When both compounds of the invention are administered once a day the specified period refers to the timing of the administration of Compound $A^2$ and Compound $B^2$ during a single day. When one or both compounds of the invention are administered more than once a day, the specified period is calculated based on the first administration of each compound on a specific day. All administrations of a compound of the invention that are subsequent to the first during a specific day are not considered when calculating the specific period.

Suitably, if the compounds are administered within a "specified period" and not administered simultaneously, they are both administered within about 24 hours of each other - in this case, the specified period will be about 24 hours; suitably they will both be administered within about 12 hours of each other - in this case, the specified period will be about 12 hours; suitably they will both be administered within about 11 hours of each other - in this case, the specified period will be about 11 hours; suitably they will both be administered within about 10 hours of each other - in this case, the specified period will be about 10 hours; suitably they will both be administered within about 9 hours of each other - in this case, the specified period will be about 9 hours; suitably they will both be administered within about 8 hours of each other - in this case, the specified period will be about 8 hours; suitably they will both be administered within about 7 hours of each other-in this case, the specified period will be about 7 hours; suitably they will both be administered within about 6 hours of each other - in this case, the specified period will be about 6 hours; suitably they will both be administered within about 5 hours of each other-in this case, the specified period will be about 5 hours; suitably they will both be administered within about 4 hours of each other - in this case, the specified period will be about 4 hours; suitably they will both be administered within about 3 hours of each other-in this case, the specified period will be about 3 hours; suitably they will be administered within about 2 hours of each other - in this case, the specified period will be about 2 hours; suitably they will both be administered within about 1 hour of each other - in this case, the specified period will be about 1 hour. As used herein, the administration of Compound $A^2$ and Compound $B^2$ in less than about 45 minutes apart is considered simultaneous administration.

Suitably, when the combination of the invention is administered for a "specified period", the compounds will be co-administered for a "duration of time".

By the term "duration of time" and derivatives thereof, as used herein is meant that both compounds of the invention are administered within a "specified period" for an indicated number of consecutive days, optionally followed by a number of consecutive days where only one of the component compounds is administered.

Regarding "specified period" administration:

Suitably, during the course of treatment, both compounds will be administered within a specified period for at least 1 day - in this case, the duration of time will be at least 1 day; suitably, during the course of treatment, both compounds will be administered within a specified period for at least 2 consecutive days - in this case, the duration of time will be at least 2 days; suitably, during the course of treatment, both compounds will be administered within a specified period for at least 3 consecutive days - in this case, the duration of time will be at least 3 days; suitably, during the course of treatment, both compounds will be administered within a specified period for at least 5 consecutive days - in this case, the duration of time will be at least 5 days; suitably, during the course of treatment, both compounds will be administered within a specified period for at least 7 consecutive days - in this case, the duration of time will be at least 7 days; suitably, during the course of treatment, both compounds will be administered within a specified period for at least 14 consecutive days - in this case, the duration of time will be at least 14 days; suitably, during the course of treatment, both compounds will be administered within a specified period for at least 30 consecutive days - in this case, the duration of time will be at least 30 days. When, during the course of treatment, both compounds are administered within a specified period for over 30 days, the treatment is considered chronic treatment and will continue until an altering event, such as a reassessment in cancer status or a change in the condition of the patient, warrants a modification to the protocol.

Further regarding "specified period" administration:

Suitably, during the course of treatment, both compounds will be administered within a specified period for at least 1 day, followed by the administration of Compound $A^2$ alone for at least 1 day - in this case, the duration of time will be at least 2 days; suitably, during the course of treatment, both compounds will be administered within a specified period for at least 1 day, followed by administration of Compound $A^2$ alone for at least 2 days - in this case, the duration

of time will be at least 3 days; suitably, during the course of treatment, both compounds will be administered within a specified period for at least 1 day, followed by administration of Compound $A^2$ alone for at least 3 days - in this case, the duration of time will be at least 4 days; suitably, during the course of treatment, both compounds will be administered within a specified period for at least 1 day, followed by administration of Compound $A^2$ alone for at least 4 days - in this case, the duration of time will be at least 5 days; suitably, during the course of treatment, both compounds will be administered within a specified period for at least 1 day, followed by administration of Compound $A^2$ alone for at least 5 days - in this case, the duration of time will be at least 6 days; suitably, during the course of treatment, both compounds will be administered within a specified period for at least 1 day, followed by administration of Compound $A^2$ alone for at least 6 days - in this case, the duration of time will be at least 7 days; suitably, during the course of treatment, both compounds will be administered within a specified period for at least 1 day, followed by administration of Compound $A^2$ alone for at least 7 days - in this case, the duration of time will be at least 8 days; suitably, during the course of treatment, both compounds will be administered within a specified period for at least 2 consecutive days, followed by administration of Compound $A^2$ alone for at least 1 day - in this case, the duration of time will be at least 3 days; suitably, during the course of treatment, both compounds will be administered within a specified period for at least 2 consecutive days, followed by administration of Compound $A^2$ alone for at least 2 consecutive days - in this case, the duration of time will be at least 4 days; suitably, during the course of treatment, both compounds will be administered within a specified period for at least 2 consecutive days, followed by administration of Compound $A^2$ alone for at least 3 consecutive days - in this case, the duration of time will be at least 5 days; suitably, during the course of treatment, both compounds will be administered within a specified period for at least 2 consecutive days, followed by administration of Compound $A^2$ alone for at least 4 consecutive days - in this case, the duration of time will be at least 6 days; suitably, during the course of treatment, both compounds will be administered within a specified period for at least 2 consecutive days, followed by administration of Compound $A^2$ alone for at least 5 consecutive days - in this case, the duration of time will be at least 7 days; suitably, during the course of treatment, both compounds will be administered within a specified period for at least 2 consecutive days, followed by administration of Compound $A^2$ alone for at least 6 consecutive days - in this case, the duration of time will be at least 8 days; suitably, during the course of treatment, both compounds will be administered within a specified period for at least 2 consecutive days, followed by administration of Compound $A^2$ alone for at least 7 consecutive days - in this case, the duration of time will be at least 9 days; suitably, during the course of treatment, both compounds will be administered within a specified period for at least 3 consecutive days, followed by administration of Compound $A^2$ alone for at least 1 day - in this case, the duration of time will be at least 4 days; suitably, during the course of treatment, both compounds will be administered within a specified period for at least 3 consecutive days, followed by administration of Compound $A^2$ alone for at least 2 consecutive days - in this case, the duration of time will be at least 5 days; suitably, during the course of treatment, both compounds will be administered within a specified period for at least 3 consecutive days, followed by administration of Compound $A^2$ alone for at least 3 consecutive days- in this case, the duration of time will be at least 6 days; suitably, during the course of treatment, both compounds will be administered within a specified period for at least 3 consecutive days, followed by administration of Compound $A^2$ alone for at least 4 consecutive days - in this case, the duration of time will be at least 7 days; suitably, during the course of treatment, both compounds will be administered within a specified period for at least 3 consecutive days, followed by administration of Compound $A^2$ alone for at least 5 consecutive days - in this case, the duration of time will be at least 8 days; suitably, during the course of treatment, both compounds will be administered within a specified period for at least 3 consecutive days, followed by administration of Compound $A^2$ alone for at least 6 consecutive days - in this case, the duration of time will be at least 9 days; suitably, during the course of treatment, both compounds will be administered within a specified period for at least 3 consecutive days, followed by administration of Compound $A^2$ alone for at least 7 consecutive days - in this case, the duration of time will be at least 10 days; suitably, during the course of treatment, both compounds will be administered within a specified period for at least 4 consecutive days, followed by administration of Compound $A^2$ alone for at least 1 day - in this case, the duration of time will be at least 5 consecutive days; suitably, during the course of treatment, both compounds will be administered within a specified period for at least 4 consecutive days, followed by administration of Compound $A^2$ alone for at least 2 consecutive days - in this case, the duration of time will be at least 6 consecutive days; suitably, during the course of treatment, both compounds will be administered within a specified period for at least 4 consecutive days, followed by administration of Compound $A^2$ alone for at least 3 consecutive days - in this case, the duration of time will be at least 7 consecutive days; suitably, during the course of treatment, both compounds will be administered within a specified period for at least 4 consecutive days, followed by administration of Compound $A^2$ alone for at least 4 consecutive days - in this case, the duration of time will be at least 8 consecutive days; suitably, during the course of treatment, both compounds will be administered within a specified period for at least 4 consecutive days, followed by administration of Compound $A^2$ alone for at least 7 consecutive days - in this case, the duration of time will be at least 11 consecutive days; suitably, during the course of treatment, both compounds will be administered within a specified period for at least 5 consecutive days, followed by administration of Compound $A^2$ alone for at least 1 day - in this case, the duration of time will be at least 6 consecutive days; suitably, during the course of treatment, both compounds will be administered within a specified period for at least 5 consecutive

days, followed by administration of Compound $A^2$ alone for at least 2 consecutive days - in this case, the duration of time will be at least 7 consecutive days; suitably, during the course of treatment, both compounds will be administered within a specified period for at least 5 consecutive days, followed by administration of Compound $A^2$ alone for at least 3 consecutive days - in this case, the duration of time will be at least 8 consecutive days; suitably, during the course of treatment, both compounds will be administered within a specified period for at least 5 consecutive days, followed by administration of Compound $A^2$ alone for at least 4 consecutive days - in this case, the duration of time will be at least 9 consecutive days; suitably, during the course of treatment, both compounds will be administered within a specified period for at least 5 consecutive days, followed by administration of Compound $A^2$ alone for at least 5 consecutive days - in this case, the duration of time will be at least 10 consecutive days; suitably, during the course of treatment, both compounds will be administered within a specified period for at least 7 consecutive days, followed by administration of Compound $A^2$ alone for at least 2 consecutive days - in this case, the duration of time will be at least 9 consecutive days; suitably, during the course of treatment, both compounds will be administered within a specified period for at least 14 consecutive days, followed by administration of Compound $A^2$ alone for at least 7 consecutive days - in this case, the duration of time will be at least 21 consecutive days; suitably, during the course of treatment, both compounds will be administered within a specified period for at least 30 consecutive days, followed by administration of Compound $A^2$ alone for at least 7 consecutive days - in this case, the duration of time will be at least 37 consecutive days. Suitably, during the course of treatment, both compounds will be administered within a specified period for from 1 to 3 consecutive days, followed by administration of Compound $A^2$ alone for from 3 to 7 consecutive days. Suitably, during the course of treatment, both compounds will be administered within a specified period for from 3 to 6 consecutive days, followed by administration of Compound $A^2$ alone for from 1 to 4 consecutive days. Suitably, during the course of treatment, both compounds will be administered within a specified period for 5 consecutive days, followed by administration of Compound $A^2$ alone for 2 consecutive days. Suitably, during the course of treatment, both compounds will be administered within a specified period for 2 consecutive days, followed by administration of Compound $A^2$ alone for from 3 to 7 consecutive days.

Further regarding "specified period" administration:

Suitably, during the course of treatment, both compounds will be administered within a specified period for at least 1 day, followed by the administration of Compound $B^2$ alone for at least 1 day - in this case, the duration of time will be at least 2 days; suitably, during the course of treatment, both compounds will be administered within a specified period for at least 1 day, followed by administration of Compound $B^2$ alone for at least 2 days - in this case, the duration of time will be at least 3 days; suitably, during the course of treatment, both compounds will be administered within a specified period for at least 1 day, followed by administration of Compound $B^2$ alone for at least 3 days - in this case, the duration of time will be at least 4 days; suitably, during the course of treatment, both compounds will be administered within a specified period for at least 1 day, followed by administration of Compound $B^2$ alone for at least 4 days - in this case, the duration of time will be at least 5 days; suitably, during the course of treatment, both compounds will be administered within a specified period for at least 1 day, followed by administration of Compound $B^2$ alone for at least 5 days - in this case, the duration of time will be at least 6 days; suitably, during the course of treatment, both compounds will be administered within a specified period for at least 1 day, followed by administration of Compound $B^2$ alone for at least 6 days - in this case, the duration of time will be at least 7 days; suitably, during the course of treatment, both compounds will be administered within a specified period for at least 1 day, followed by administration of Compound $B^2$ alone for at least 7 days - in this case, the duration of time will be at least 8 days; suitably, during the course of treatment, both compounds will be administered within a specified period for at least 2 consecutive days, followed by administration of Compound $B^2$ alone for at least 1 day - in this case, the duration of time will be at least 3 days; suitably, during the course of treatment, both compounds will be administered within a specified period for at least 2 consecutive days, followed by administration of Compound $B^2$ alone for at least 2 consecutive days - in this case, the duration of time will be at least 4 days; suitably, during the course of treatment, both compounds will be administered within a specified period for at least 2 consecutive days, followed by administration of Compound $B^2$ alone for at least 3 consecutive days - in this case, the duration of time will be at least 5 days; suitably, during the course of treatment, both compounds will be administered within a specified period for at least 2 consecutive days, followed by administration of Compound $B^2$ alone for at least 4 consecutive days - in this case, the duration of time will be at least 6 days; suitably, during the course of treatment, both compounds will be administered within a specified period for at least 2 consecutive days, followed by administration of Compound $B^2$ alone for at least 5 consecutive days - in this case, the duration of time will be at least 7 days; suitably, during the course of treatment, both compounds will be administered within a specified period for at least 2 consecutive days, followed by administration of Compound $B^2$ alone for at least 6 consecutive days - in this case, the duration of time will be at least 8 days; suitably, during the course of treatment, both compounds will be administered within a specified period for at least 2 consecutive days, followed by administration of Compound $B^2$ alone for at least 7 consecutive days - in this case, the duration of time will be at least 9 days; suitably, during the course of treatment, both compounds will be administered within a specified period for at least 3 consecutive days, followed by administration of Compound $B^2$ alone for at least 1 day - in this case, the duration of time will be at least 4 days; suitably, during the

course of treatment, both compounds will be administered within a specified period for at least 3 consecutive days, followed by administration of Compound $B^2$ alone for at least 2 consecutive days - in this case, the duration of time will be at least 5 days; suitably, during the course of treatment, both compounds will be administered within a specified period for at least 3 consecutive days, followed by administration of Compound $B^2$ alone for at least 3 consecutive days- in this case, the duration-of time will be at least 6 days; suitably, during the course of treatment, both compounds will be administered within a specified period for at least 3 consecutive days, followed by administration of Compound $B^2$ alone for at least 4 consecutive days - in this case, the duration of time will be at least 7 days; suitably, during the course of treatment, both compounds will be administered within a specified period for at least 3 consecutive days, followed by administration of Compound $B^2$ alone for at least 5 consecutive days - in this case, the duration of time will be at least 8 days; suitably, during the course of treatment, both compounds will be administered within a specified period for at least 3 consecutive days, followed by administration of Compound $B^2$ alone for at least 6 consecutive days - in this case, the duration of time will be at least 9 days; suitably, during the course of treatment, both compounds will be administered within a specified period for at least 3 consecutive days, followed by administration of Compound $B^2$ alone for at least 7 consecutive days - in this case, the duration of time will be at least 10 days; suitably, during the course of treatment, both compounds will be administered within a specified period for at least 4 consecutive days, followed by administration of Compound $B^2$ alone for at least 1 day - in this case, the duration of time will be at least 5 consecutive days; suitably, during the course of treatment, both compounds will be administered within a specified period for at least 4 consecutive days, followed by administration of Compound $B^2$ alone for at least 2 consecutive days - in this case, the duration of time will be at least 6 consecutive days; suitably, during the course of treatment, both compounds will be administered within a specified period for at least 4 consecutive days, followed by administration of Compound $B^2$ alone for at least 3 consecutive days - in this case, the duration of time will be at least 7 consecutive days; suitably, during the course of treatment, both compounds will be administered within a specified period for at least 4 consecutive days, followed by administration of Compound $B^2$ alone for at least 4 consecutive days - in this case, the duration of time will be at least 8 consecutive days; suitably, during the course of treatment, both compounds will be administered within a specified period for at least 4 consecutive days, followed by administration of Compound $B^2$ alone for at least 7 consecutive days - in this case, the duration of time will be at least 11 consecutive days; suitably, during the course of treatment, both compounds will be administered within a specified period for at least 5 consecutive days, followed by administration of Compound $B^2$ alone for at least 1 day - in this case, the duration of time will be at least 6 consecutive days; suitably, during the course of treatment, both compounds will be administered within a specified period for at least 5 consecutive days, followed by administration of Compound $B^2$ alone for at least 2 consecutive days - in this case, the duration of time will be at least 7 consecutive days; suitably, during the course of treatment, both compounds will be administered within a specified period for at least 5 consecutive days, followed by administration of Compound $B^2$ alone for at least 3 consecutive days - in this case, the duration of time will be at least 8 consecutive days; suitably, during the course of treatment, both compounds will be administered within a specified period for at least 5 consecutive days, followed by administration of Compound $B^2$ alone for at least 4 consecutive days - in this case, the duration of time will be at least 9 consecutive days; suitably, during the course of treatment, both compounds will be administered within a specified period for at least 5 consecutive days, followed by administration of Compound $B^2$ alone for at least 5 consecutive days - in this case, the duration of time will be at least 10 consecutive days; suitably, during the course of treatment, both compounds will be administered within a specified period for at least 7 consecutive days, followed by administration of Compound $B^2$ alone for at least 2 consecutive days - in this case, the duration of time will be at least 9 consecutive days; suitably, during the course of treatment, both compounds will be administered within a specified period for at least 14 consecutive days, followed by administration of Compound $B^2$ alone for at least 7 consecutive days - in this case, the duration of time will be at least 21 consecutive days; suitably, during the course of treatment, both compounds will be administered within a specified period for at least 30 consecutive days, followed by administration of Compound $B^2$ alone for at least 7 consecutive days - in this case, the duration of time will be at least 37 consecutive days. Suitably, during the course of treatment, both compounds will be administered within a specified period for from 1 to 3 consecutive days, followed by administration of Compound $B^2$ alone for from 3 to 7 consecutive days. Suitably, during the course of treatment, both compounds will be administered within a specified period for from 3 to 6 consecutive days, followed by administration of Compound $B^2$ alone for from 1 to 4 consecutive days. Suitably, during the course of treatment, both compounds will be administered within a specified period for 5 consecutive days, followed by administration of Compound $B^2$ alone for 2 consecutive days. Suitably, during the course of treatment, both compounds will be administered within a specified period for 2 consecutive days, followed by administration of Compound $B^2$ alone for from 3 to 7 consecutive days.

Further regarding "specified period" administration:

Suitably, during the course of treatment, Compound $A^2$ and Compound $B^2$ will be administered within a specified period for from 1 to 3 days over a 7 day period, and during the other days of the 7 day period Compound $A^2$ will be administered alone. Suitably, this 7 day protocol is repeated for 2 cycles or for 14 days; suitably for 4 cycles or 28 days; suitably for continuous administration.

Suitably, during the course of treatment, Compound $A^2$ and Compound $B^2$ will be administered within a specified period for from 1 to 3 days over a 7 day period, and during the other days of the 7 day period Compound $B^2$ will be administered alone. Suitably, this 7 day protocol is repeated for 2 cycles or for 14 days; suitably for 4 cycles or 28 days; suitably for continuous administration.

Suitably, during the course of treatment, Compound $A^2$ and Compound $B^2$ will be administered within a specified period for 3 days over a 7 day period, and during the other days of the 7 day period Compound $A^2$ will be administered alone. Suitably, this 7 day protocol is repeated for 2 cycles or for 14 days; suitably for 4 cycles or 28 days; suitably for continuous administration.

Suitably, during the course of treatment, Compound $A^2$ and Compound $B^2$ will be administered within a specified period for 3 days over a 7 day period, and during the other days of the 7 day period Compound $B^2$ will be administered alone. Suitably, this 7 day protocol is repeated for 2 cycles or for 14 days; suitably for 4 cycles or 28 days; suitably for continuous administration.

Suitably, during the course of treatment, Compound $A^2$ and Compound $B^2$ will be administered within a specified period for 2 days over a 7 day period, and during the other days of the 7 day period Compound $A^2$ will be administered alone. Suitably, this 7 day protocol is repeated for 2 cycles or for 14 days; suitably for 4 cycles or 28 days; suitably for continuous administration.

Suitably, during the course of treatment, Compound $A^2$ and Compound $B^2$ will be administered within a specified period for 2 days over a 7 day period, and during the other days of the 7 day period Compound $B^2$ will be administered alone. Suitably, this 7 day protocol is repeated for 2 cycles or for 14 days; suitably for 4 cycles or 28 days; suitably for continuous administration.

Suitably, during the course of treatment, Compound $A^2$ and Compound $B^2$ will be administered within a specified period for 1 day during a 7 day period, and during the other days of the 7 day period Compound $A^2$ will be administered alone. Suitably, this 7 day protocol is repeated for 2 cycles or for 14 days; suitably for 4 cycles or 28 days; suitably for continuous administration.

Suitably, during the course of treatment, Compound $A^2$ and Compound $B^2$ will be administered within a specified period for 1 day during a 7 day period, and during the other days of the 7 day period Compound $B^2$ will be administered alone. Suitably, this 7 day protocol is repeated for 2 cycles or for 14 days; suitably for 4 cycles or 28 days; suitably for continuous administration.

Suitably, during the course of treatment, Compound $A^2$ and Compound $B^2$ will be administered within a specified period for from 1 to 5 days over a 14 day period, and during the other days of the 14 day period Compound $A^2$ will be administered alone. Suitably, this 14 day protocol is repeated for 2 cycles or for 28 days; suitably for continuous administration.

Suitably, during the course of treatment, Compound $A^2$ and Compound $B^2$ will be administered within a specified period for from 1 to 5 days over a 14 day period, and during the other days of the 14 day period Compound $B^2$ will be administered alone. Suitably, this 14 day protocol is repeated for 2 cycles or for 28 days; suitably for continuous administration.

Suitably, if the compounds are not administered during a "specified period", they are administered sequentially. By the term "sequential administration", and derivates thereof, as used herein is meant that one of Compound $A^2$ and Compound $B^2$ is administered once a day for two or more consecutive days and the other of Compound $A^2$ and Compound $B^2$ is subsequently administered once a day for two or more consecutive days. Also, contemplated herein is a drug holiday utilized between the sequential administration of one of Compound $A^2$ and Compound $B^2$ and the other of Compound $A^2$ and Compound $B^2$. As used herein, a drug holiday is a period of days after the sequential administration of one of Compound $A^2$ and Compound $B^2$ and before the administration of the other of Compound $A^2$ and Compound $B^2$ where neither Compound $A^2$ nor Compound $B^2$ is administered. Suitably the drug holiday will be a period of days selected from: 1 day, 2 days, 3 days, 4 days, 5 days, 6 days, 7 days, 8 days, 9 days, 10 days, 11 days, 12 days, 13 days and 14 days.

Suitably, if the compounds are not administered during a "specified period", they are administered sequentially. By the term "sequential administration", and derivates thereof, as used herein is meant that one of Compound $A^2$ and Compound $B^2$ is administered for one or more consecutive days and the other of Compound $A^2$ and Compound $B^2$ is subsequently administered for one or more consecutive days.

Regarding sequential administration:

Suitably, one of Compound $A^2$ and Compound $B^2$ is administered for from 2 to 30 consecutive days, followed by an optional drug holiday, followed by administration of the other of Compound $A^2$ and Compound $B^2$ for from 2 to 30 consecutive days. Suitably, one of Compound $A^2$ and Compound $B^2$ is administered for from 2 to 21 consecutive days, followed by an optional drug holiday, followed by administration of the other of Compound $A^2$ and Compound $B^2$ for from 2 to 21 consecutive days. Suitably, one of Compound $A^2$ and Compound $B^2$ is administered for from 2 to 14 consecutive

days, followed by a drug holiday of from 1 to 14 days, followed by administration of the other of Compound $A^2$ and Compound $B^2$ for from 2 to 14 consecutive days. Suitably, one of Compound $A^2$ and Compound $B^2$ is administered for from 3 to 7 consecutive days, followed by a drug holiday of from 3 to 10 days, followed by administration of the other of Compound $A^2$ and Compound $B^2$ for from 3 to 7 consecutive days.

Suitably, one of Compound $A^2$ and Compound $B^2$ is administered for from 1 to 30 consecutive days, followed by an optional drug holiday, followed by administration of the other of Compound $A^2$ and Compound $B^2$ for from 1 to 30 consecutive days. Suitably, one of Compound $A^2$ and Compound $B^2$ is administered for from 1 to 21 consecutive days, followed by an optional drug holiday, followed by administration of the other of Compound $A^2$ and Compound $B^2$ for from 1 to 21 consecutive days. Suitably, one of Compound $A^2$ and Compound $B^2$ is administered for from 1 to 14 consecutive days, followed by a drug holiday of from 1 to 14 days, followed by administration of the other of Compound $A^2$ and Compound $B^2$ for from 1 to 14 consecutive days. Suitably, one of Compound $A^2$ and Compound $B^2$ is administered for from 2 to 7 consecutive days, followed by a drug holiday of from 2 to 10 days, followed by administration of the other of Compound $A^2$ and Compound $B^2$ for from 2 to 7 consecutive days.

Suitably, Compound $B^2$ will be administered first in the sequence, followed by an optional drug holiday, followed by administration of Compound $A^2$. Suitably, Compound $B^2$ is administered for from 3 to 21 consecutive days, followed by an optional drug holiday, followed by administration of Compound $A^2$ for from 3 to 21 consecutive days. Suitably, Compound $B^2$ is administered for from 3 to 21 consecutive days, followed by a drug holiday of from 1 to 14 days, followed by administration of Compound $A^2$ for from 3 to 21 consecutive days. Suitably, Compound $B^2$ is administered for from 3 to 21 consecutive days, followed by a drug holiday of from 3 to 14 days, followed by administration of Compound $A^2$ for from 3 to 21 consecutive days. Suitably, Compound $B^2$ is administered for 21 consecutive days, followed by an optional drug holiday, followed by administration of Compound $A^2$ for 14 consecutive days. Suitably, Compound $B^2$ is administered for 14 consecutive days, followed by a drug holiday of from 1 to 14 days, followed by administration of Compound $A^2$ for 14 consecutive days. Suitably, Compound $B^2$ is administered for 7 consecutive days, followed by a drug holiday of from 3 to 10 days, followed by administration of Compound $A^2$ for 7 consecutive days. Suitably, Compound $B^2$ is administered for 3 consecutive days, followed by a drug holiday of from 3 to 14 days, followed by administration of Compound A for 7 consecutive days. Suitably, Compound $B^2$ is administered for 3 consecutive days, followed by a drug holiday of from 3 to 10 days, followed by administration of Compound $A^2$ for 3 consecutive days.

Suitably, Compound $B^2$ will be administered first in the sequence, followed by an optional drug holiday, followed by administration of Compound $A^2$. Suitably, Compound $B^2$ is administered for from 1 to 21 consecutive days, followed by an optional drug holiday, followed by administration of Compound $A^2$ for from 1 to 21 consecutive days. Suitably, Compound $B^2$ is administered for from 3 to 21 consecutive days, followed by a drug holiday of from 1 to 14 days, followed by administration of Compound $A^2$ for from 3 to 21 consecutive days. Suitably, Compound $B^2$ is administered for from 3 to 21 consecutive days, followed by a drug holiday of from 3 to 14 days, followed by administration of Compound $A^2$ for from 3 to 21 consecutive days. Suitably, Compound $B^2$ is administered for 21 consecutive days, followed by an optional drug holiday, followed by administration of Compound $A^2$ for 14 consecutive days. Suitably, Compound $B^2$ is administered for 14 consecutive days, followed by a drug holiday of from 1 to 14 days, followed by administration of Compound $A^2$ for 14 consecutive days. Suitably, Compound $B^2$ is administered for 7 consecutive days, followed by a drug holiday of from 3 to 10 days, followed by administration of Compound $A^2$ for 7 consecutive days. Suitably, Compound $B^2$ is administered for 3 consecutive days, followed by a drug holiday of from 3 to 14 days, followed by administration of Compound $A^2$ for 7 consecutive days. Suitably, Compound $B^2$ is administered for 3 consecutive days, followed by a drug holiday of from 3 to 10 days, followed by administration of Compound $A^2$ for 3 consecutive days.

Suitably, Compound $A^2$ will be administered first in the sequence, followed by an optional drug holiday, followed by administration of Compound $B^2$. Suitably, Compound $A^2$ is administered for from 1 to 21 consecutive days, followed by an optional drug holiday, followed by administration of Compound $B^2$ for from 1 to 21 consecutive days. Suitably, Compound $A^2$ is administered for from 3 to 21 consecutive days, followed by a drug holiday of from 1 to 14 days, followed by administration of Compound $B^2$ for from 3 to 21 consecutive days. Suitably, Compound $A^2$ is administered for from 3 to 21 consecutive days, followed by a drug holiday of from 3 to 14 days, followed by administration of Compound $B^2$ for from 3 to 21 consecutive days. Suitably, Compound $A^2$ is administered for 21 consecutive days, followed by an optional drug holiday, followed by administration of Compound $B^2$ for 14 consecutive days. Suitably, Compound $A^2$ is administered for 14 consecutive days, followed by a drug holiday of from 1 to 14 days, followed by administration of Compound $B^2$ for 14 consecutive days. Suitably, Compound $A^2$ is administered for 7 consecutive days, followed by a drug holiday of from 3 to 10 days, followed by administration of Compound $B^2$ for 7 consecutive days. Suitably, Compound $A^2$ is administered for 3 consecutive days, followed by a drug holiday of from 3 to 14 days, followed by administration of Compound $B^2$ for 7 consecutive days. Suitably, Compound $A^2$ is administered for 3 consecutive days, followed by a drug holiday of from 3 to 10 days, followed by administration of Compound $B^2$ for 3 consecutive days. Suitably, Compound $A^2$ is administered for 7 consecutive days, followed by administration of Compound $B^2$ for 1 day. Suitably, Compound $A^2$ is administered for 6 consecutive days, followed by administration of Compound $B^2$ for 1 day. Suitably, Compound $B^2$ is administered for 1 day, followed by administration of Compound $A^2$ for 7 consecutive days. Suitably, Compound

$B^2$ is administered for 1 day, followed by administration of Compound $A^2$ for 6 consecutive days.

It is understood that a "specified period" administration and a "sequential" administration can be followed by repeat dosing or can be followed by an alternate dosing protocol, and a drug holiday may precede the repeat dosing or alternate dosing protocol.

Suitably, the amount of Compound $A^2$ administered as part of the combination according to the present invention will be an amount selected from about 0.125mg to about 10mg; suitably, the amount will be selected from about 0.25mg to about 9mg; suitably, the amount will be selected from about 0.25mg to about 8mg; suitably, the amount will be selected from about 0.5mg to about 8mg; suitably, the amount will be selected from about 0.5mg to about 7mg; suitably, the amount will be selected from about 1 mg to about 7mg; suitably, the amount will be about 5mg. Accordingly, the amount of Compound A administered as part of the combination according to the present invention will be an amount selected from about 0.125mg to about 10 mg. For example, the amount of Compound $A^2$ administered as part of the combination according to the present invention can be 0.125mg, 0.25mg, 0.5mg, 0.75mg, 1mg, 1.5mg, 2mg, 2.5mg, 3mg, 3.5mg, 4mg, 4.5mg, 5mg, 5.5mg, 6mg, 6.5mg, 7mg, 7.5mg, 8mg, 8.5mg, 9mg, 9.5mg, 10mg. Suitably, the selected amount of Compound $A^2$ is administered twice a day. Suitably, the selected amount of Compound $A^2$ is administered once a day. Suitably, the administration of Compound $A^2$ will begin as a loading dose. Suitably, the loading dose will be an amount from 2 to 100 times the maintenance dose; suitably from 2 to 10 times; suitably from 2 to 5 times; suitably 2 times; suitably 3 times; suitably 4 times; suitably 5 times. Suitably, the loading does will be administered from 1 to 7 days; suitably from 1 to 5 days; suitably from 1 to 3 days; suitably for 1 day; suitably for 2 days; suitably for 3 days, followed by a maintenance dosing protocol.

Suitably, the amount of Compound $B^2$ administered as part of the combination according to the present invention will be an amount selected from about 5mg to about 500mg; suitably, the amount will be selected from about 25mg to about 400mg; suitably, the amount will be selected from about 30mg to about 375mg; suitably, the amount will be selected from about 35mg to about 350mg; suitably, the amount will be selected from about 40mg to about 300mg; suitably, the amount will be selected from about 45mg to about 275mg; suitably, the amount will be selected from about 50mg to about 250mg; suitably, the amount will be selected from about 55mg to about 225mg; suitably, the amount will be selected from about 60mg to about 200mg; suitably, the amount will be selected from about 65mg to about 175mg; suitably, the amount will be selected from about 70mg to about 150mg; suitably, the amount will be selected from about 50mg to about 300mg; suitably, the amount will be selected from about 75mg to about 150mg; suitably, the amount will be about 100mg. Accordingly, the amount of Compound $B^2$ administered as part of the combination according to the present invention will be an amount selected from about 5mg to about 500mg. For example, the amount of Compound $B^2$ administered as part of the combination according to the present invention can be 5mg, 10mg, 15mg, 20mg, 25mg, 30mg, 35mg, 40mg, 45mg, 50mg, 55mg, 60mg, 65mg, 70mg, 75mg, 80mg, 85mg, 90mg, 95mg, 100mg, 105mg, 110mg, 115mg, 120mg, 125mg, 130mg, 135mg, 140mg, 145mg, 150mg, 175mg, 200mg, 225mg, 250mg, 275mg, 300mg, 325mg, 350mg, 375mg, 400mg, 425mg, 450mg, 475mg or 500mg. Suitably, the selected amount of Compound $B^2$ is administered twice a day. Suitably, the selected amount of Compound $B^2$ is administered once a day. Suitably, the administration of Compound $B^2$ will begin as a loading dose. Suitably, the loading dose will be an amount from 2 to 100 times the maintenance dose; suitably from 2 to 10 times; suitably from 2 to 5 times; suitably 2 times; suitably 3 times; suitably 4 times; suitably 5 times. Suitably, the loading does will be administered from 1 to 7 days; suitably from 1 to 5 days; suitably from 1 to 3 days; suitably for 1 day; suitably for 2 days; suitably for 3 days, followed by a maintenance dosing protocol.

Suitably, the amount of Compound $B^2$ administered as part of the combination according to the present invention will be an amount selected from about 75mg to about 1,000mg; suitably, the amount will be selected from about 100mg to about 900mg; suitably, the amount will be selected from about 150mg to about 850mg; suitably, the amount will be selected from about 200mg to about 800mg; suitably, the amount will be selected from about 250mg to about 750mg; suitably, the amount will be selected from about 300mg to about 6000mg; suitably, the amount will be about 450mg. Accordingly, the amount of Compound $B^2$ administered as part of the combination according to the present invention will be an amount selected from about 75mg to about 1,000mg. For example, the amount of Compound $B^2$ administered as part of the combination according to the present invention can be 75mg, 100 mg, 125mg, 150 mg, 175mg, 200mg, 225mg, 250mg, 275mg, 300mg, 325mg, 350mg, 375mg, 400mg, 425mg, 450mg, 475mg, 500mg, 525mg, 550mg, 575mg, 600mg, 625mg, 650mg, 675mg, 700mg, 725mg, 750mg, 775mg, 800mg, 825mg, 850mg, 875mg, 900mg, 925mg, 950mg, 975mg or 1,000mg.

As used herein, all amounts specified for Compound $A^2$ and Compound $B^2$ are indicated as the administered amount of free or unsalted and unsolvated compound per dose.

The combination of the present invention may also be employed with other therapeutic compounds in the cancer treatment.

While it is possible that, for use in therapy, therapeutically effective amounts of the combinations of the present invention may be administered as the raw chemical, it is preferable to present the combinations as a pharmaceutical composition or compositions. Accordingly, the invention further provides pharmaceutical compositions, which include

Compound $A^2$ and/or Compound $B^2$, and one or more pharmaceutically acceptable carriers. The combinations of the present invention are as described above. The carrier(s) must be acceptable in the sense of being compatible with the other ingredients of the formulation, capable of pharmaceutical formulation, and not deleterious to the recipient thereof. In accordance with another aspect of the invention there is also provided a process for the preparation of a pharmaceutical formulation including admixing Compound $A^2$ and/or Compound $B^2$ with one or more pharmaceutically acceptable carriers. As indicated above, such elements of the pharmaceutical combination utilized may be presented in separate pharmaceutical compositions or formulated together in one pharmaceutical formulation.

Pharmaceutical formulations may be presented in unit dose forms containing a predetermined amount of active ingredient per unit dose. As is known to those skilled in the art, the amount of active ingredient per dose will depend on the condition being treated, the route of administration and the age, weight and condition of the patient. Preferred unit dosage formulations are those containing a daily dose or sub-dose, or an appropriate fraction thereof, of an active ingredient. Furthermore, such pharmaceutical formulations may be prepared by any of the methods well known in the pharmacy art.

Compound $A^2$ and Compound $B^2$ may be administered by any appropriate route. Suitable routes include oral, rectal, nasal, topical (including buccal and sublingual), vaginal, and parenteral (including subcutaneous, intramuscular, intravenous, intradermal, intrathecal, and epidural). It will be appreciated that the preferred route may vary with, for example, the condition of the recipient of the combination and the cancer to be treated. It will also be appreciated that each of the agents administered may be administered by the same or different routes and that Compound $A^2$ and Compound $B^2$ may be compounded together in a pharmaceutical composition/formulation. Suitably, Compound $A^2$ and Compound $B^2$ are administered in separate oral pharmaceutical compositions.

The compounds or combinations of the current invention are incorporated into convenient dosage forms such as capsules, tablets, or injectable preparations. Solid or liquid pharmaceutical carriers are employed. Solid carriers include, starch, lactose, calcium sulfate dihydrate, terra alba, sucrose, talc, gelatin, agar, pectin, acacia, magnesium stearate, and stearic acid. Liquid carriers include syrup, peanut oil, olive oil, saline, and water. Similarly, the carrier may include a prolonged release material, such as glyceryl monostearate or glyceryl distearate, alone or with a wax. The amount of solid carrier varies widely but, preferably, will be from about 25 mg to about 1 g per dosage unit. When a liquid carrier is used, the preparation will suitably be in the form of a syrup, elixir, emulsion, soft gelatin capsule, sterile injectable liquid such as an ampoule, or an aqueous or nonaqueous liquid suspension.

For instance, for oral administration in the form of a tablet or capsule, the active drug component can be combined with an oral, non-toxic pharmaceutically acceptable inert carrier such as ethanol, glycerol, water. Powders are prepared by comminuting the compound to a suitable fine size and mixing with a similarly comminuted pharmaceutical carrier such as an edible carbohydrate, as, for example, starch or mannitol. Flavoring, preservative, dispersing and coloring agent can also be present.

It should be understood that in addition to the ingredients mentioned above, the formulations may include other agents conventional in the art having regard to the type of formulation in question, for example those suitable for oral administration may include flavoring agents.

As indicated, therapeutically effective amounts of the combinations of the invention (Compound $A^2$ in combination with Compound $B^2$) are administered to a human. Typically, the therapeutically effective amount of the administered agents of the present invention will depend upon a number of factors including, for example, the age and weight of the subject, the precise condition requiring treatment, the severity of the condition, the nature of the formulation, and the route of administration. Ultimately, the therapeutically effective amount will be at the discretion of the attendant physician.

The combinations of the present invention are tested for efficacy, advantageous and synergistic properties according to known procedures.

Suitably, the combinations of the invention are tested for efficacy, advantageous and synergistic properties generally according to the following combination cell proliferation assays. Cells are plated in 384-well plates at 500 cells/well in culture media appropriate for each cell type, supplemented with 10% FBS and 1% penicillin/streptomycin, and incubated overnight at 37°C, 5% $CO_2$. Cells are treated in a grid manner with dilution of Compound $A^2$ (20 dilutions, including no compound, of 2-fold dilutions starting from 1-20 $\mu$M depending of compound) from left to right on 384-well plate and also treated with Compound $B^2$ (20 dilutions, including no compound, of 2-fold dilutions starting from 1-20 $\mu$M depending of compound) from top to bottom on 384-well plate and incubated as above for a further 72 hours. In some instances compounds are added in a staggered manner and incubation time can be extended up to 7days. Cell growth is measured using CellTiter-Glo® reagent according to the manufacturer's protocol and signals are read on a PerkinElmer EnVision™ reader set for luminescence mode with a 0.5-second read. Data are analyzed as described below.

Results are expressed as a percentage of the t=0 value and plotted against compound(s) concentration. The t=0 value is normalized to 100% and represents the number of cells present at the time of compound addition. The cellular response is determined for each compound and/or compound combination using a 4- or 6-parameter curve fit of cell viability against concentration using the IDBS XLfit plug-in for Microsoft Excel software and determining the concentration required for 50% inhibition of cell growth ($gIC_{50}$). Background correction is made by subtraction of values

from wells containing no cells. For each drug combination a Combination Index (CI), Excess Over Highest Single Agent (EOHSA) and Excess Over Bliss (EOBliss) are calculated according to known methods such as described in Chou and Talalay (1984) Advances in Enzyme Regulation, 22, 37 to 55; and Berenbaum, MC (1981) Adv. Cancer Research, 35, 269-335.

**In vitro cell growth inhibition by Compound A, Compound B and their combination in tumor cell lines**

**Methods:**

**Analysis of Breast cancer cell lines**

**Cell lines and growth conditions**

Human breast tumor lines, BT-474, HCC1419, HCC1937, HCC1954, HCC202, KPL-1, MDA-MB-157, MDA-MB-175-VII, MDA-MB-361, MDA-MB-453, SK-BR-3, SUM225PE, UACC893, and ZR-75-1; lung tumor line, CALU-3; and melanoma line, CHL-1, were from ATCC. Human breast tumor line JIMT-1 was from European Collection of Cell Cultures (UK). Human breast tumor lines SUM149PT, SUM190PT and SUM52PE were from Asterand. These lines were cultured in RPMI 1640 medium containing 10 % fetal bovine serum (FBS). A head and neck tumor line, LICR LON HN5 (HN5), a gift from the Institute of Cancer Research, Surrey, U.K, was cultured in Dulbecco's modified Eagle medium (DMEM) containing 5% FBS; KPL-4 breast tumor cell line was kindly provided by Dr Junichi Kurebayashi (Kawasaki Medical School, Okayama, Japan) and cultured in DMEM containing 5% FBS. JIMT-1 is a line derived from a patient clinically resistant to trastuzumab (Herceptin®). BT-474-J4 is a single cell clone derived from a pool of BT-474 cells that were selected to grow in lapatinib to a concentration of 3 $\mu$M.

**Cell growth inhibition assay and combination data analysis.**

All cells were cultured for a minimum of 72 hours prior to cell plating. Cells were assayed in a 96-well tissue culture plate (NUNC 136102) of RPMI medium containing 10% FBS for all cells at 2,000 cells per well except KPL-4 and HN5 which were plated in DMEM containing 5% FBS at 500 cells per well. Approximately 24 hours after plating, cells were exposed to ten, two-fold or three-fold serial dilutions of compound or the combination of the two agents at a constant molar to molar ratio of 1:1 Compound A to Compound B in RPMI media containing 10% FBS or DMEM containing 5% FBS. Cells were incubated in the presence of compounds for 3 days. ATP levels were determined by adding Cell Titer Glo® (Promega) according to the manufacturer's protocol. Briefly, Cell Titer Glo® was added each plate, incubated for 20 minutes then luminescent signal was read on the SpectraMax L plate reader with a 0.5 sec integration time.

Inhibition of cell growth was estimated after treatment with compound or combination of compounds for three days and comparing the signal to cells treated with vehicle (DMSO). Cell growth was calculated relative to vehicle (DMSO) treated control wells. Concentration of compound that inhibits 50% of control cell growth ($IC_{50}$) was interpolated using nonlinear regression with the equation, y=(A+(B-A)/(1+(C/x)^D))), where A is the minimum response ($y_{min}$), B is the maximum response ($y_{max}$), C is the inflection point of the curve ($EC_{50}$) and D is the Hill coefficient.

Combination effects on potency were evaluated using Combination Index (CI) which was calculated with the back-interpolated $IC_{50}$ values and the mutually non-exclusive equation derived by Chou and Talalay (1): $CI = Da/IC_{50}(a) + Db/IC_{50}(b) + (Da \times Db)/(IC_{50}(a) \times IC_{50}(b))$
where $IC_{50}(a)$ is the $IC_{50}$ of Compound A; $IC_{50}(b)$ is the $IC_{50}$ for Compound B; Da is the concentration of Compound A in combination with Compound B that inhibited 50 % of cell growth; and Db is the concentration of Compound B in combination with Compound A that inhibited 50% of cell growth. In general, a CI value <0.9, between 0.9 and 1.1, or >1.1 indicates synergy, additivity and antagonism, respectively. In general, the smaller the CI number, the greater is the strength of synergy.

The combination effects on the response scale were quantified by Excess Over Highest Single Agent (EOHSA) based on the concept of nonlinear blending as described in detail by Peterson and Novick (2007) and Peterson (2010) [(2;3) [Peterson and Novick, 2007; Peterson, 2010]. EOHSA values are defined as increases in improvement (here, in 'percentage points' (ppts) difference) produced by the combination over the best single agent at its component dose level for the combination. For single agent and combination treatments, cells were exposed to compounds at a fixed-dose-ratio, and dose response curves were fit to the experimental data and analyzed using regression models. At specified total dose levels of $IC_{50}$ along the dose response curve, the dose combination (corresponding to $IC_{50}$) was determined for making EOHSA statistical inferences. More specifically, for a combination drug experiment involving drug 1 at dose d1 and drug 2 at dose d2, (i.e., total dose equals d1 +d2) is said to have a positive EOHSA if the mean response at the combination is better than the mean response to drug 1 at dose d1 or drug 2 at dose d2.

Results of the analysis of breast cancer cell lines are reported in Table 1 and Figure 1 below.

## Colon, Lung and Pancreas Cell Line Proliferation Study

In a separate study, combination drug tests with Compounds A and B were conducted using a panel of cell lines from human colon cancers (n = 26), lung cancers (n = 15) and pancreatic cancers (n = 6). Cell lines were obtained from commercial vendors (ATCC and DSMZ). Cell lines were grown in RPMI-1640 supplemented with 2 mM glutamine, 1mM sodium pyruvate and 10% fetal bovine serum (except for Capan-1 and HuP-T4 which were grown with 20% fetal bovine serum) and maintained at 37°C and 5%$CO_2$ in a humid incubator. Assays were performed in 384 well microtiter plates with optimum seeding densities for each cell line.

The test compounds were prepared as 10 mM stocks in 100% DMSO. Further dilutions of the compounds were made in DMSO. Compound A was diluted horizontally in a separate 96 well microtiter plate in rows D-G using a 3-fold dilution series for 10 dilution points. Compound B was similarly diluted horizontally in a 96 well microtiter plate in rows B-E using a 3-fold dilution series for 10 dilution points.

The two compounds were combined using equal volumes from each drug plate into cell culture media. This resulted in a 1:50 dilution of the drugs. Both Compound A and Compound B were individually titrated in rows B and C (for Compound B) and rows F and G (for Compound A) of the merged drug plate.

A 1:10 dilution of the drugs was performed in cell culture media prior to addition to the cells. Drug addition to the cells resulted in a further 1:2 dilution of drugs for a total dilution of 1:1000.

The final concentration range for the test compounds was 250 to 0.013 nM for Compound A and 1000 to 0.5 nM for Compound B. The positive control consisted of culture media with DMSO at 0.1% and cells. The negative control consisted of culture media with DMSO at 0.1%. The cell lines were incubated at 37°C, 5% $CO_2$ in humid air for 72 hours. Cell proliferation was measured using the CellTiter Glo (Promega) reagent according to the manufacturer's protocol. The plates are treated with CellTiter Glo solution and are analyzed for RLU (relative light units) using a Molecular Devices SpectraMax M5 plate reader.

## Data Analysis

Three independent metrics were used to analyze the combinatorial effects on growth inhibition of Compound B and Compound A.

The percent intensity values were used in model 205 of XLfit ( IDBS, Inc.) in Microsoft Excel to calculate $gIC_{50}$ values using a 4 parameter logistical fit. The midpoint of the growth window (the $gIC_{50}$) falls half way between the number of cells at the time of compound addition (T=0) and the growth of control cells treated with DMSO at 72 hrs. The number of cells at time zero ($T_0$) is divided from the intensity value at the bottom of the response curve ($Y_{min}$) to generate a measure for cell death ($Y_{min}/T_0$). A value below 1 for $Y_{min}/T_0$ indicates stronger potency to induce cell death with the treatment when compared to higher values.

1. Excess over Highest Single Agent (EOHSA) - This was calculated as described above (Borisy et al, 2003; FDA 21 CFR 300.50)

2. Bliss synergy - A second criterion often used to determine combination synergy is evaluating the excess inhibition over Bliss independence or "additivity" (Bliss and Mexico, 1939). The model assumes a combined response of the two compounds independently using the following:

$$E_a + E_b - (E_a * E_b)$$

Where $E_a$ is the effect (or percent inhibition) of Compound A and $E_b$ is the effect of Compound B. The resulting effect of the combination of the two compounds is compared to their predicted additivity by Bliss and a synergy score is generated for each dose along the response curve.

3. Combination Index (CI)- A third criterion traditionally used for the evaluation of synergy is Combination Index (CI) derived from the Chou and Talalay (1984). The following equation is a model used for compounds that behave with different mechanisms of action (mutually non-exclusive formula). This was calculated as described above.

For EOHSA and Bliss Synergy measures, a score is generated for each dose along the response curve. These scores reflect the percentage over the highest agent (EOHSA) or percentage greater than Bliss additivity, depending on which metric is being interpreted. The scores across the entire dose curve are evaluated and those combinations that show high scores (>10) in the therapeutic concentration range for both replicates are considered to be synergistic.

The higher the score, the greater the effect of the combination for the two compounds. For the Combination Index, the lower the CI, the more synergy is seen with the combination.

For those cell lines that never reached an inhibitory concentration of 25% for 1 of the compounds in the combination, a CI value cannot be calculated and no value is listed for the CI in Tables 4, 7 and 10.

A subset of the cell lines were analyzed in duplicate (colon: n = 4; lung: n = 13; pancreas: n = 3). For all subsequent analyses, data for these cell lines was averaged.

**Cell Line Mutation Data**

Mutation data was collated for the status of selected cancer related genes. The data source is the cancer cell line mutation screening data published as part of the Catalog of Somatic Mutations in Cancer database (COSMIC) (Bamford S. et al. Br. J. Cancer. 2004. 91:355-58). In order to ensure that the identity of the cell lines used in the proliferation assay matched that in the COSMIC database, a genotype comparison was done between those cell lines in the sensitivity screen and those in COSMIC. Specifically, this entailed:

1. Calculating the genotypes for each cell line using the Affymetrix 500K 'SNP Chip' (Affymetrix, Inc., Sunnyvale, CA) and the RLMM algorithm (Rabbee & Speed, Bioinformatics, 2006. 22: 7-12).

2. Identifying the genotype matches of each cell line to those pre-calculated for each cell line having mutation profiles in COSMIC.

3. Assigning mutation status for each cell line in based upon the genotype matches.

**Results:**

**Breast, Melanoma, Head and Neck, and Lung Cancer Cell Panel**

The effect of cell growth inhibition by a mitogen activated protein/ERK-kinase (MEK) inhibitor Compound A, an AKT inhibitor Compound B, and their combination was determined in a panel of human tumor cell lines. The mean $IC_{50}$s (from at least two independent experiments) and the combination effects at $IC_{50}$s are summarized in Table 1. Representative dose response curves for MDA-MB-175-VII, BT-474-J4 and JIMT-1 cell lines are provided in Figure 1. A subset of breast cancer cell lines including HER2 gene amplification (HER2+) lines KPL-4, UACC893, SUM190PT, HCC1954 and MDA-MB-453 with PIK3CA_H1047R mutation, MDA-MB-361 with PIK3CA_E545K mutation, and SUM225PE with wild type PIK3CA; and non-HER2+ lines ZR-75-1, SUM52PE and MDA-MB-175-VII were sensitive to single agent Compound B with $IC_{50}$ < 1 $\mu$M. On the other hand, breast tumor lines MDA-MB-175-VII and SUM149PT; head and neck line, HN5; lung line Calu3; and melanoma line, CHL-1, were sensitive to single agent Compound A($IC_{50}$ < 1$\mu$M). However all the cell lines listed in Table 1 were more sensitive to the combination of Compound A and Compound B as indicated by their reduced $IC_{50}$ values ranging from 0.01 to 0.76 $\mu$M. The combination of Compound A and Compound B showed cell growth inhibition more than the most active single agent alone as demonstrated by EOHSA values of 8-39 ppt in all lines (Table 1), and synergistic with combination index (CI) value of 0.34 in MDA-MB-175-VII, a line sensitive to either Compound A or Compound B (Figure 1-A). BT-474-J4, a derivative from the BT-474 parental line that shows an increased level of resistance to lapatinib, displayed increased sensitivity to Compound B as a single agent ($IC_{50}$ = 0.271 $\mu$M) compared to BT-474 parental cells ($IC_{50}$ >1 $\mu$M). The combination of Compound A and Compound B showed a benefit of enhanced cell growth inhibition in BT-474-J4 with an EOSHA value of 25 ppt (Figure 1-B). JIMT-1, a cell line derived from a patient that progressed on trastuzumab was not sensitive to either Compound A or Compound B as single agents. Combining Compound A and Compound B was beneficial in JIMT-1 cells with an EOSHA value of 27 ppt (Figure 1-C).

**Colon, Lung and Pancreas Cell Line Panel**

The effect of cell growth inhibition by a mitogen activated protein/ERK-kinase (MEK) inhibitor Compound A, an AKT inhibitor Compound B, and their combination was determined in a panel of human colon (n =26), lung (n = 15), and pancreas (n = 6) cell lines. A summary of these results are presented in Tables 2 and 3. In colon cancers, 77% (20/26) showed synergy by at least one metric. Additionally, all colon cancer cell lines (26/26) showed an increase in cell killing (as measured by the change in $Y_{min}$ over the highest single agent); while 7/26 (27%) showed an increase > 20%. Lung lines had high rates of synergy, where 11/15 (73%) showed synergy by at least one metric. A total of 7/15 (47%) cell lines showed an increase of cell killing > 20%. Pancreatic cell lines also showed high rates of synergistic growth inhibition, where 4/6 (67%) showed synergy by at least one unique metric. Similarly 4/6 (67%) demonstrated an increase in cell killing > 20% over the highest single agent.

Table 1. Cell growth inhibition by Compound A, Compound B and their combination in human tumor cell lines.

| Tumor Type | Cell Line | Gene Amp+ | PIK3CA and PTEN Status | $IC_{50}$ values in micromolar (mean +/- std) | | | | Combination Effect |
|---|---|---|---|---|---|---|---|---|
| | | | | Single Agent | | Equal Molar Ratio Combination | | |
| | | | | Compound A | Compound B | Compound A | Compound B | EOHSA (ppt) |
| Breast | KPL-4 | HER2+ | PIK3CA H1047R | >1 | 0.017 +/- 0.003 | 0.010 +/- 0.003 | 0.010 +/- 0.003 | 13 +/- 9 |
| | UACC893 | HER2+ | PIK3CA H1047R | >1 | 0.070 +/- 0.066 | 0.014 +/- 0.011 | 0.014 +/- 0.011 | 34 +/- 6 |
| | SUM190PT | HER2+ | PIK3CA H1047R | >1 | 0.112 +/- 0.005 | 0.013 +/- 0.003 | 0.013 +/- 0.003 | 17 +/- 6 |
| | HCC1954 | HER2+ | PIK3CA H1047R | >1 | 0.412 +/- 0.180 | 0.042 +/- 0.023 | 0.042 +/- 0.023 | 22 +/- 12 |
| | MDA-MB-453 | HER2+ | PIK3CA H1047R | >1 | 0.366 +/- 0.013 | 0.106 +/- 0.022 | 0.106 +/- 0.022 | 29 +/- 1 |
| | MDA-MB-361 | HER2+ | PIK3CA E545K | >1 | 0.169 +/- 0.055 | 0.106 +/- 0.025 | 0.106 +/- 0.025 | 15 +/- 4 |
| | BT-474-J4 | HER2+ | PIK3CA K111N | >1 | 0.217 +/- 0.198 | 0.036 +/- 0.018 | 0.036 +/- 0.018 | 25 +/- 9 |
| | SUM225PE | HER2+ | WT | >1 | 0.529 +/- 0.402 | 0.178 +/- 0.154 | 0.178 +/- 0.154 | 20 +/- 0 |
| | HCC1419 | HER2+ | WT | >1 | >1 | 0.280 +/- 0.209 | 0.280 +/- 0.209 | 23 +/- 8 |
| | BT-474 | HER2+ | PIK3CA K111N | >1 | >1 | 0.659 +/- 0.597 | 0.659 +/- 0.597 | 13 +/- 4 |
| | HCC202 | HER2+ | PIK3CA E545K | >1 | >1 | 0.187 +/- 0.102 | 0.187 +/- 0.102 | 33 +/- 8 |
| | JimT-1 | HER2+ | PIK3CA C420R | >1 | >1 | 0.255 +/- 0.099 | 0.255 +/- 0.099 | 27 +/- 8 |
| | SK-BR-3 | HER2+ | WT | >1 | >1 | 0.759 +/- 0.266 | 0.759 +/- 0.266 | 12 +/- 3 |
| | ZR-75-1 | | PTEN L108R | >1 | 0.042 +/- 0.005 | 0.032 +/- 0.001 | 0.032 +/- 0.001 | 8 +/- 2 |
| | SUM52PE | FGFR2+ | WT | >1 | 0.230 +/- 0.109 | 0.036 +/- 0.040 | 0.036 +/- 0.040 | 19 +/- 23 |
| | MDA-MB-175-VII | | WT | 0.063 +/- 0.002 | 0.137 +/- 0.001 | 0.014 +/- 0.001 | 0.014 +/- 0.001 | 23 +/- 2 |
| | SUM149PT | | PTEN low | 0.279 +/- 0.358 | >1 | 0.024 +/- 0.006 | 0.024 +/- 0.006 | 22 +/- 2 |
| | KPL-1 | | PIK3CA E545K | >1 | >1 | 0.178 +/- 0.012 | 0.178 +/- 0.012 | 39 +/- 2 |
| | HCC1937 | | PTEN low | >1 | >1 | 0.373 +/- 0.101 | 0.373 +/- 0.101 | 25 +/- 1 |
| | MDA-MB-157 | | WT | >1 | >1 | 0.757 +/- 0.147 | 0.757 +/- 0.147 | 21 +/- 8 |
| H&N | HN5 | EGFR+ | WT | 0.301 +/- 0.137 | >1 | 0.059 +/- 0.008 | 0.059 +/- 0.008 | 25 +/- 8 |
| Lung | Calu-3 | HER2+ | WT | 0.085 +/- 0.012 | >1 | 0.044 +/- 0.009 | 0.044 +/- 0.009 | 11 +/- 6 |
| Melanoma | CHL-1 | | WT | 0.417 +/- 0.164 | >1 | 0.068 +/- 0.013 | 0.068 +/- 0.013 | 16 +/- 6 |

HER2+: HER2 gene amplified; EGFR+: EGFR gene amplified, FGFR2+: FGFR2 gene amplified.

Cell growth inhibition-dose response curves for MDA-MB-175-VII, BT474-J4 and JIMT-1 are depicted in Figure 1 below.

Reference List

(1) Chou TC, Talalay P. Quantitative analysis of dose-effect relationships: the combined effects of multiple drugs or enzyme inhibitors. Adv Enzyme Regul 1984;22:27-55.

(2) Peterson JJ, Novick SJ. Nonlinear blending: a useful general concept for the assessment of combination drug synergy. J Recept Signal Transduct Res 2007;27(2-3):125-46.

(3) Peterson J. A Review of Synergy Concepts of Nonlinear Blending and Dose-Reduction Profiles. Frontiers of Bioscience S2, 483-503. 2010.

**Table 2.** Panel of pancreatic (n=6), colon (n=26) and lung (n=15) cell lines and mutation status used for combination studies.

| Cell Line | Organ Site | Diagnosis/Histology | KRAS | NRAS | BRAF | PIK3CA | PTEN |
|---|---|---|---|---|---|---|---|
| NCI-H747 | Colon | Adenocarcinoma | p.G13D | WT | WT | WT | WT |
| LS1034 | Colon | Adenocarcinoma | p.A146T | WT | WT | WT | WT |
| SW948 | Colon | Adenocarcinoma | p.Q61L | WT | WT | p.E542K | WT |
| LS174T | Colon | Adenocarcinoma | p.G12D | WT | WT | p.H1047R | WT |
| SW116 | Colon | Adenocarcinoma | p.G12A | WT | WT | WT | WT |
| T84 | Colon | Carcinoma | p.G13D | WT | WT | p.E542K | WT |
| Colo 201 | Colon | Adenocarcinoma | WT | WT | p.V600E | WT | WT |
| SW403 | Colon | Carcinoma | p.G12V | WT | WT | WT | WT |
| DLD-1 | Colon | Carcinoma | p.G13D | WT | WT | p.E545K | WT |
| Colo205 | Colon | Adenocarcinoma | p.G12V | WT | p.V600E | WT | WT |
| Colo 320HSR | Colon | Adenocarcinoma | WT | WT | WT | WT | WT |
| SW620 | Colon | Adenocarcinoma | p.G12V | WT | WT | WT | WT |
| NCI-H508 | Colon | Adenocarcinoma | WT | WT | WT | p.E545K | WT |
| Colo-320DM | Colon | Adenocarcinoma | Unavail | Unavail | Unavail | Unavail | Unavail |
| SW837 | Colon | Adenocarcinoma | p.G12C | WT | WT | WT | WT |
| KM12 | Colon | Adenocarcinoma | WT | WT | WT | WT | p.G129*,p.K267fs*9 |
| WiDr | Colon | Adenocarcinoma | WT | WT | p.V600E | p.P449T | WT |
| HCT-8 | Colon | ileocecal colorectal adenocarcinoma | p.G13D | WT | WT | p.E545K | WT |
| RKO | Colon | Carcinoma | WT | WT | p.V600E | p.H1047R | WT |
| HT-29 | Colon | Carcinoma | WT | WT | p.V600E | p.P449T | WT |
| SW480 | Colon | Adenocarcinoma | p.G12V | WT | WT | WT | WT |
| HCT-15 | Colon | Adenocarcinoma | p.G13D | WT | WT | p.E545K | WT |
| HCT116 | Colon | Carcinoma | p.G13D | WT | WT | p.H1047R | WT |
| SW48 | Colon | Adenocarcinoma | WT | WT | WT | WT | WT |
| SW1417 | Colon | Adenocarcinoma | WT | WT | p.V600E | WT | WT |
| HCC2998 | Colon | Carcinoma | p.A146T | WT | WT | WT | WT |
| Calu-6 | Lung | Adenocarcinoma | p.Q61K | WT | WT | WT | WT |
| SK-MES-1 | Lung | Squamous cell carcinoma | WT | WT | WT | WT | WT |
| A549 | Lung | Alveoloar basal epithelial-squamous | p.G12S | WT | WT | WT | WT |
| NCI-H2170 | Lung | Squamous cell carcinoma | WT | WT | WT | WT | WT |
| NCI-H2228 | Lung | Adenocarcinoma | WT | WT | WT | WT | WT |
| NCI-H23 | Lung | Adenocarcinoma | WT | WT | p.V600E | p.P449T | WT |
| NCI-H1792 | Lung | Adenocarcinoma | p.G12C | WT | WT | WT | WT |
| NCI-H358 | Lung | Branchio-alveolar | p.G12C | WT | WT | WT | WT |

| | | | | | | | |
|---|---|---|---|---|---|---|---|
| **NCI-H2122** | Lung | Adenocarcinoma | p.G12C | WT | WT | WT | WT |
| **NCI-H520** | Lung | Squamous cell carcinoma | WT | WT | WT | WT | WT |
| **NCI-H1299** | Lung | Non-small cell lung cancer | WT | p.Q61K | WT | WT | WT |
| **NCI-H1563** | Lung | Adenocarcinoma | WT | WT | WT | WT | WT |
| **NCI-H460** | Lung | Large cell carcinoma | p.Q61H | WT | WT | p.E545K | WT |
| **NCI-H2030** | Lung | Adenocarcinoma | p.G12C | WT | WT | WT | WT |
| **SW900** | Lung | Carcinoma | p.G12V | WT | WT | WT | WT |
| **BxPC-3** | Pancreas | Adenocarcinoma | WT | WT | WT | WT | WT |
| **SW1990** | Pancreas | Adenocarcinoma | p.G12D | WT | WT | WT | WT |
| **YAPC** | Pancreas | Carcinoma | p.G12V | WT | WT | WT | WT |
| **Mia PaCa** | Pancreas | Carcinoma | p.G12C | WT | WT | WT | WT |
| **HPAFII** | Pancreas | Carcinoma | p.G12D | WT | WT | WT | WT |
| **ASPC1** | Pancreas | Carcinoma | p.G12D | WT | WT | WT | WT |

**Table 2 key**
**Cell Line = Cell line name**
**Organ Site = Organ from which cells were derived**
**Diagnosis/Histology = Pathological diagnosis of tissue**
**KRAS/NRAS/BRAF/PIK3CA/PTEN = Mutation status; WT = Wild Type; Unavail = Data not available**

Table 3. Cell growth inhibition by Compound A, Compound B and their combination in human colon, lung and pancreatic cell lines. GIC50 values are presented in nM.

| Cell Lines | | Compound A | | Compound B | | Combination | | Differential over Highest Single Agent | | Synergy Metrics | | |
|---|---|---|---|---|---|---|---|---|---|---|---|---|
| Cell Line | Tumor Type | $gIC_{50}$ (nM) | $Y_{min}/T_0$ | $gIC_{50}$(nM) | $Y_{min}/T_0$ | $gIC_{50}$ (nM) | $Y_{min}/T_0$ | $Y_{min}$ (%) | $gIC_{50}$ (nM) | EOHSA | BLISS | Comb Index |
| Colo201 | Colon | 4.0 | -8.96 | >1000.00 | 80.18 | 106.78 | 19.02 | -10.06 | 106.78 | Additive | Synergy | |
| Colo205 | Colon | 0.77 | -1.30 | >1000.00 | 14.87 | 25.81 | -7.30 | -6.01 | 25.81 | Modest | Modest | |
| Colo320DM | Colon | >250.00 | 81.01 | >1000.00 | 9.81 | >1000.00 | 7.71 | -2.10 | >1000.0 | No Synergy | Modest | |
| Colo320HS R | Colon | >250.00 | 78.4 | >1000.00 | 26.6 | >1000.00 | 23.0 | -4.9 | >1000.0 | Modest | Synergy | |
| DLD1 | Colon | 342.3 | 39.7 | >1000.00 | 29.0 | 310.8 | 9.1 | -19.9 | 310.8 | Synergy | Modest | |
| HCC2998 | Colon | 16.7 | 2.13 | 292.3 | -4.90 | 17.55 | 11.08 | -6.17 | 17.55 | Synergy | Synergy | Synergy |
| HCT116 | Colon | 23.2 | 19.40 | >1000.00 | 25.31 | 137.65 | 0.51 | -18.89 | 137.65 | Synergy | Synergy | |
| HCT15 | Colon | >250.00 | 57.0 | >1000.00 | 30.4 | 781.1 | 9.6 | -20.8 | 781.1 | Modest | Modest | |
| HCT8 | Colon | 13.30 | 16.70 | 758.1 | 20.44 | 112.72 | -1.09 | -17.80 | 112.72 | Synergy | Synergy | Synergy |
| HT29 | Colon | 3.6 | 11.99 | 780.5 | 11.64 | 65.01 | -3.14 | -14.78 | 65.01 | Modest | Synergy | Synergy |
| KM12 | Colon | 28.7 | 19.1 | 927.6 | 13.9 | 154.4 | 1.3 | -12.7 | 154.4 | Synergy | Synergy | Synergy |
| LS1034 | Colon | 36.99 | 2.87 | >1000.00 | -0.67 | 328.82 | 14.71 | -14.05 | 328.82 | Synergy | Synergy | |
| LS174T | Colon | 81.4 | 18.28 | >1000.00 | 19.70 | 599.62 | 0.17 | -18.10 | 599.62 | Synergy | Synergy | |
| NCIH508 | Colon | 22.64 | 15.22 | 68.2 | 11.12 | 16.23 | 5.72 | -5.40 | 16.23 | Synergy | Synergy | Synergy |
| NCIH747 | Colon | 5.35 | 5.55 | >1000.00 | 23.79 | 52.90 | 10.08 | -15.63 | 52.90 | Modest | Synergy | |
| RKO | Colon | 77.8 | 24.44 | >1000.00 | 17.13 | 106.33 | -5.73 | -22.86 | 106.33 | Synergy | Synergy | |
| SW1116 | Colon | 14.61 | 2.28 | >1000.00 | 37.32 | 164.38 | 22.08 | -24.36 | 164.38 | Modest | Modest | |
| SW1417 | Colon | 2.86 | 16.19 | >1000.00 | 3.63 | 31.62 | 14.56 | -18.19 | 31.62 | Modest | Modest | |
| SW403 | Colon | 4.6 | 3.02 | 74.3 | 8.82 | 25.95 | 12.74 | -15.76 | 25.95 | Synergy | Additive | Synergy |
| SW48 | Colon | 8.16 | 13.02 | 428.4 | 11.68 | 16.92 | 14.72 | -3.04 | 16.92 | Synergy | Modest | Synergy |
| SW480 | Colon | 325.65 | 29.37 | >1000.00 | 11.87 | 105.87 | 10.73 | -22.60 | 105.87 | Synergy | Modest | |
| SW620 | Colon | 15.2 | 15.46 | >1000.00 | 76.65 | 261.31 | 4.76 | -10.71 | 261.31 | Modest | Modest | |
| SW837 | Colon | 153.4 | 25.58 | >1000.00 | 63.88 | 658.09 | -3.94 | -29.52 | 558.09 | Synergy | Synergy | |
| SW948 | Colon | 185.2 | 27.87 | 609.3 | 20.46 | 267.82 | 11.48 | -31.94 | 267.82 | Synergy | Synergy | Synergy |
| WiDr | Colon | 1.85 | 3.94 | 700.4 | 5.24 | 28.50 | -6.92 | -10.86 | 28.50 | Synergy | Modest | Synergy |
| T84 | Colon | 138.64 | 12.44 | 427.4 | -5.58 | 105.11 | 27.17 | -21.59 | 105.11 | Synergy | Modest | Synergy |
| A549 | Lung | 12.9 | 15.9 | >1000.00 | 32.7 | 147.7 | -5.4 | -21.3 | 147.7 | Synergy | Modest | |
| Calu6 | Lung | 53.1 | 15.0 | >1000.00 | 56.3 | 868.0 | -4.4 | -19.4 | 868.0 | Modest | Additive | |
| NCIH1299 | Lung | 24.0 | 45.9 | >1000.00 | 58.1 | 295.1 | 20.2 | -25.7 | 295.1 | Synergy | Modest | |

(continued)

| Cell Lines | | Compound A | | Compound B | | Combination | | Differential over Highest Single Agent | | Synergy Metrics | | |
|---|---|---|---|---|---|---|---|---|---|---|---|---|
| Cell Line | Tumor Type | $gIC_{50}$ (nM) | $Y_{min}/T_0$ | $gIC_{50}$(nM) | $Y_{min}/T_0$ | $gIC_{50}$ (nM) | $Y_{min}$ /$T_0$ | $Y_{min}$ (%) | $gIC_{50}$ (nM) | EOHSA | BLISS | Comb Index |
| NCIH1563 | Lung | >250.00 | 26.6 | 65.0 | 27.6 | 91.2 | -22.1 | -48.6 | 91.2 | Synergy | Modest | |
| NCIH1792 | Lung | 42.0 | 2.8 | >1000.00 | 26.0 | 234.1 | -14.4 | -17.2 | 234.1 | Synergy | Modest | |
| NCIH2030 | Lung | >250.00 | 55.8 | >1000.00 | 40.7 | 440.0 | 3.3 | -17.2 | 440.0 | Synergy | Modest | |
| NCIH2122 | Lung | 30.0 | -11.8 | >1000.00 | 11.4 | 266.5 | -15.5 | -3.7 | 266.5 | Synergy | Synergy | |
| NCIH2170 | Lung | >250.00 | 59.5 | >1000.00 | 18.2 | 512.2 | -0.2 | -18.3 | 512.2 | Synergy | Synergy | |
| NCIH2228 | Lung | >250.00 | 39.7 | >1000.00 | 37.1 | 357.5 | -6.6 | -43.7 | 357.5 | Synergy | Synergy | |
| NCIH23 | Lung | >250.00 | 36.3 | >1000.00 | 41.5 | 540.9 | -1.9 | -38.1 | 540.9 | Synergy | Synergy | |
| NCIH358 | Lung | 17.3 | 10.7 | >1000.00 | 37.2 | 83.8 | -10.7 | -21.4 | 83.8 | Modest | Additive | |
| NCIH460 | Lung | 63.0 | 39.1 | 664.2 | 20.8 | 151.1 | 3.2 | -17.6 | 151.1 | Synergy | Modest | Modest |
| NCIH520 | Lung | >250.00 | 70.2 | 568.4 | 15.0 | 450.7 | 2.6 | -12.4 | 450.7 | Additive | Modest | |
| SKMES1 | Lung | 80.4 | 19.5 | >1000.00 | 49.6 | 604.5 | -20.1 | -39.6 | 604.5 | Synergy | Synergy | |
| SW900 | Lung | 15.0 | -5.9 | >1000.00 | 47.3 | 149.1 | -24.3 | -18.4 | 149.1 | Modest | Modest | |
| ASPC1 | Pancreas | 19.7 | 13.6 | >1000.00 | 42.9 | 44.3 | -8.2 | -21.8 | 44.3 | Additive | Additive | |
| BxPC3 | Pancreas | >250.00 | 20.89 | >1000.00 | 71.87 | 360.17 | - 25.90 | -46.78 | 360.17 | Synergy | Synergy | |
| HPAFII | Pancreas | 15.9 | -0.8 | >1000.00 | 31.2 | 95.4 | -14.5 | -13.7 | 95.4 | Synergy | Synergy | |
| MlaPaCa | Pancreas | 23.5 | 25.0 | >1000.00 | 0.7 | 430.9 | 10.1 | 9.4 | 430.9 | Modest | Modest | |
| SW1990 | Pancreas | 94.0 | 17.1 | >1000.00 | 36.8 | 412.4 | -8.4 | -25.6 | 412.4 | Synergy | Modest | |
| YAPC | Pancreas | >250.00 | 59.4 | >1000.00 | 55.2 | 641.3 | 17.9 | -37.4 | 641.3 | Synergy | Modest | |

Table 3 Key:
Cell Line = Tumor-derived cell line
$gIC_{50}$ = Concentration of compound required to cause 50% growth inhibition
$Y_{min}$ (%)= Percent of the minimum cellular growth in the presence of Compound B (relative to DMSO control) as measured by % of that at T=0 (number of cells at time of Compound B addition). A negative number indicates a net loss of cells relative to that at T=0.
$Y_{min}$ /$T_0$ = $Y_{min}$ divided by the number of cells at time zero
EOHSA= Excess over highest single agent determination
BLISS = Bliss synergy determination
Comb Index = Combination Index score

**Effect of Compound B (AKT inhibitor) in combination with Compound A (MEK inhibitor) on the growth of human pancreatic tumor xenografts (HPAC and Capan2) in SCID mice**

**Method:**

Female SCID mice were implanted subcutaneously with HPAC or Capan2 tumor cells (human pancreatic carcinoma harboring mutant KRAS gene). When the tumor volume reached ~150 $mm^3$, mice were block randomized to different treatment groups (n=8 mice/group). Mice received AKT inhibitor, Compound B, at 10 or 30 mg/kg, once daily (QD). MEK inhibitor, Compound A, was administered at 0.1, 0.3 and 1.0 mg/kg, once daily (QD) alone or at 0.1 and 0.3 mg/kg once daily in combination with AKT inhibitor. Mice were weighed and tumors measured by calipers twice weekly. Treatment was continued till the tumor volume reached >1000 $mm^3$. Tumor volumes were calculated using the formula: tumor volume = (Length x $Width^2$)/2. The percentage of tumor growth inhibition was calculated on each day of tumor measurement using the formula: 100x[1-(average growth of the compound-treated tumors / average growth of vehicle-treated control tumors)]. Data is plotted as mean $\pm$ sem for tumor volume for each group.

**Results:**

Treatment of mice bearing HPAC tumors with Compound B showed minimal inhibition (11-15%) in 10 mg/kg group and modest inhibition (31-40%) in 30 mg/kg group in two independent studies. Montherapy with Compound A showed a dose dependent inhibition of HPAC tumor growth ~40, 60 and 90% growth inhibition at 0.1, 0.3 and 1 mg/kg, respectively. In Capan2 xenograft model, Compound B showed a similar growth inhibition (27-30%) at both 10 and 30 mg/kg doses, whereas administration of Compound A at 0.1, 0.3 and 1.0 mg/kg resulted in 70, 87 and 104% growth inhibition, respectively. Combined treatment with both AKT inhibitor (Compound B) and MEK inhibitor (Compound A) resulted in increased anti-tumor activity compared to either agent alone at the respective doses for both HPAC and Capan2 tumor xenografts (data summarized in Table 4 and Figure 2).

**Table 4: Inhibition of HPAC and Capan2 tumor xenograft growth in mice treated with Compound B and Compound A**

| | | % Tumor Growth Inhibition | | |
|---|---|---|---|---|
| **Group** | **Regimen** | **HPAC-D01** | **HPAC-D02** | **Capan2-D01** |
| | **Treatment Duration** | **75** | **74** | **56** |
| #1 | Vehicle/Control | -- | -- | |
| #2 | Compound A 0.1 mg/kg po, QD | 38% | 41% | 74% |
| #3 | Compound A 0.3mg/kg po, QD | 60% | 65% | 90% |
| #4 | Compound A 1 mg/kg po, QD | 91% | 89% | 104% |
| #5 | Compound B 10mg/kg po, QD | 11% | 15% | 22% |
| #6 | Compound B 30mg/kg po, QD | 40% | 31% | 26% |
| #7 | Compound A 0.1mg/kg + Compound B 10mg/kg po, QD | 76% | 65% | 84% |
| #8 | Compound A 0.1 mg/kg + Compound B 30mg/kg po, QD | 75% | 67% | 75% |
| #9 | Compound A 0.3mg/kg + Compound B 10mg/kg po, QD | 80% | 72% | 99% |
| #10 | Compound A 0.3mg/kg + Compound B 30mg/kg po, QD | 93% | 93% | 108% |

Because the combinations of the present invention are active in the above assays they exhibit advantageous therapeutic utility in treating cancer.

Suitably, the present invention relates to a combination for use in treating or lessening the severity of a cancer selected from: brain (gliomas), glioblastomas, astrocytomas, glioblastoma multiforme, Bannayan-Zonana syndrome, Cowden disease, Lhermitte-Duclos disease, breast, inflammatory breast cancer, Wilm's tumor, Ewing's sarcoma, Rhabdomyosarcoma, ependymoma, medulloblastoma, colon, head and neck, kidney, lung, liver, melanoma, ovarian, pancreatic, prostate, sarcoma, osteosarcoma, giant cell tumor of bone, thyroid,
Lymphoblastic T cell leukemia, Chronic myelogenous leukemia, Chronic lymphocytic leukemia, Hairy-cell leukemia, acute lymphoblastic leukemia, acute myelogenous leukemia, Chronic neutrophilic leukemia, Acute lymphoblastic T cell leukemia, Plasmacytoma, Immunoblastic large cell leukemia, Mantle cell leukemia, Multiple myeloma Megakaryoblastic leukemia, multiple myeloma, acute megakaryocytic leukemia, promyelocytic leukemia, Erythroleukemia,
malignant lymphoma, hodgkins lymphoma, non-hodgkins lymphoma, lymphoblastic T cell lymphoma, Burkitt's lymphoma, follicular lymphoma,
neuroblastoma, bladder cancer, urothelial cancer, lung cancer, vulval cancer, cervical cancer, endometrial cancer, renal cancer, mesothelioma, esophageal cancer, salivary gland cancer, hepatocellular cancer, gastric cancer, nasopharangeal cancer, buccal cancer, cancer of the mouth, GIST (gastrointestinal stromal tumor) and testicular cancer.

Suitably, the present invention relates to a combination for use in treating or lessening the severity of a cancer selected from: brain (gliomas), glioblastomas, astrocytomas, glioblastoma multiforme, Bannayan-Zonana syndrome, Cowden disease, Lhermitte-Duclos disease, breast, colon, head and neck, kidney, lung, liver, melanoma, ovarian, pancreatic, prostate, sarcoma and thyroid.

Suitably, the present invention relates to a combination for use in treating or lessening the severity of a cancer selected from ovarian, breast, pancreatic and prostate.

Suitably the present invention relates to a combination for use in treating or lessening the severity of pre-cancerous syndromes in a mammal, including a human, wherein the pre-cancerous syndrome is selected from: cervical intraepithelial neoplasia, monoclonal gammapathy of unknown significance (MGUS), myelodysplastic syndrome, aplastic anemia, cervical lesions, skin nevi (pre-melanoma), prostatic intraepithleial (intraductal) neoplasia (PIN), Ductal Carcinoma in situ (DCIS), colon polyps and severe hepatitis or cirrhosis.

Suitably, the present invention relates to a combination for use in treating or lessening the severity of a cancer that is either wild type or mutant for Ras/Raf and either wild type or mutant for PI3K/Pten. This includes patients wild type for both Ras/Raf and PI3K/PTEN, mutant for both Ras/Raf and PI3K/PTEN, mutant for Ras/Raf and wild type for PI3K/PTEN and wild type for Ras/Raf and mutant for PI3K/PTEN.

The term "wild type" as is understood in the art refers to a polypeptide or polynucleotide sequence that occurs in a native population without genetic modification. As is also understood in the art, a "mutant" includes a polypeptide or polynucleotide sequence having at least one modification to an amino acid or nucleic acid compared to the corresponding amino acid or nucleic acid found in a wild type polypeptide or polynucleotide, respectively. Included in the term mutant is Single Nucleotide Polymorphism (SNP) where a single base pair distinction exists in the sequence of a nucleic acid strand compared to the most prevalently found (wild type) nucleic acid strand.

Cancers that are either wild type or mutant for Ras/Raf and either wild type or mutant for PI3K/Pten are identified by known methods.

For example, wild type or mutant Ras/Raf or PI3K/PTEN tumor cells can be identified by DNA amplification and sequencing techniques, DNA and RNA detection techniques, including, but not limited to Northern and Southern blot, respectively, and/or various biochip and array technologies. Wild type and mutant polypeptides can be detected by a variety of techniques including, but not limited to immunodiagnostic techniques such as ELISA, Western blot or imunocyto chemistry.

This invention provides a combination comprising N-{3-[3-cyclopropyl-5-(2-fluoro-4-iodo-phenylamino)6,8-dimethyl-2,4,7-trioxo-3,4,6,7-tetrahydro-2H-pyrido[4,3-d]pyrimidin-1-yl]phenyl}acetamide, or a pharmaceutically acceptable salt or solvate thereof, and *N*-{(1*S*)-2-amino-1-[(3,4-difluorophenyl)methyl]ethyl}-5-chloro-4-(4-chloro-1-methyl-1*H*-pyrazol-5-yl)-2-furancarboxamide, or a pharmaceutically acceptable salt thereof.

This invention also provides a combination comprising N-{3-[3-cyclopropyl-5-(2-fluoro-4-iodo-phenylamino)6,8-dimethyl-2,4,7-trioxo-3,4,6,7-tetrahydro-2H-pyrido[4,3-d]pyrimidin-1-yl]phenyl}acetamide, or a pharmaceutically acceptable salt or solvate thereof, and *N*-{(1*S*)-2-amino-1-[(3,4-difluorophenyl)methyl]ethyl}-5-chloro-4-(4-chloro-1-methyl-1*H*-pyrazol-5-yl)-2-furancarboxamide, or a pharmaceutically acceptable salt thereof, for use in therapy.

This invention also provides a combination comprising N-{3-[3-cyclopropyl-5-(2-fluoro-4-iodo-phenylamino)6,8-dimethyl-2,4,7-trioxo-3,4,6,7-tetrahydro-2H-pyrido[4,3-d]pyrimidin-1-yl]phenyl}acetamide, or a pharmaceutically acceptable salt or solvate thereof, and *N*-{(1*S*)-2-amino-1-[(3,4-difluorophenyl)methyl]ethyl}-5-chloro-4-(4-chloro-1-methyl-1*H*-pyrazol-5-yl)-2-furancarboxamide, or a pharmaceutically acceptable salt thereof, for use in treating cancer.

This invention also provides a pharmaceutical composition comprising a combination of N-{3-[3-cyclopropyl-5-(2-fluoro-4-iodo-phenylamino)6,8-dimethyl-2,4,7-trioxo-3,4,6,7-tetrahydro-2H-pyrido[4,3-d]pyrimidin-1-yl]phenyl}acetamide, or a pharmaceutically acceptable salt or solvate thereof, and *N*-{(1S)-2-amino-1-[(3,4-difluorophenyl)methyl]ethyl}-5-chloro-4-(4-chloro-1-methyl-1*H*-pyrazol-5-yl)-2-furancarboxamide, or a pharmaceutically acceptable salt thereof.

This invention also provides a combination kit comprising N-{3-[3-cyclopropyl-5-(2-fluoro-4-iodo-phenylamino)6,8-dimethyl-2,4,7-trioxo-3,4,6,7-tetrahydro-2H-pyrido[4,3-d]pyrimidin-1-yl]phenyl}acetamide, or a pharmaceutically

acceptable salt or solvate thereof, and *N*-{(1*S*)-2-amino-1-[(3,4-difluorophenyl}methyl]ethyl}-5-chloro-4-(4-chloro-1-methyl-1*H*-pyrazol-5-yl)-2-furancarboxamide, or a pharmaceutically acceptable salt thereof.

This invention also provides the use of a combination comprising N-{3-[3-cyclopropyl-5-(2-fluoro-4-iodo-phenylamino)6,8-dimethyl-2,4,7-trioxo-3,4,6,7-tetrahydro-2H-pyrido[4,3-d]pyrimidin-1-yl]phenyl}acetamide, or a pharmaceutically acceptable salt or solvate thereof, and *N*-{(1S)-2-amino-1-[(3,4-difluorophenyl)methyl]ethyl}-5-chloro-4-(4-chloro-1-methyl-1*H*-pyrazol-5-yl)-2-furancarboxamide, or a pharmaceutically acceptable salt thereof, in the manufacture of a medicament.

This invention also provides the use of a combination comprising N-{3-[3-cyclopropyl-5-(2-fluoro-4-iodo-phenylamino)6,8-dimethyl-2,4,7-trioxo-3,4,6,7-tetrahydro-2H-pyrido[4,3-d]pyrimidin-1-yl]phenyl}acetamide, or a pharmaceutically acceptable salt or solvate thereof, and *N*-{(1*S*)-2-amino-1-[(3,4-difluorophenyl)methyl]ethyl}-5-chloro-4-(4-chloro-1-methyl-1*H*-pyrazol-5-yl)-2-furancarboxamide, or a pharmaceutically acceptable salt thereof, in the manufacture of a medicament to treat cancer.

## Experimental Details

### Example 1 - Capsule Composition

An oral dosage form for administering a combination of the present invention is produced by filing a standard two piece hard gelatin capsule with the ingredients in the proportions shown in Table I, below.

Table I

| INGREDIENTS | AMOUNTS |
|---|---|
| N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide (the dimethyl sulfoxide solvate of Compound A) | 5mg |
| *N*-{(1*S*)-2-amino-1-[(3,4-difluorophenyl)methyl]ethyl}-5-chloro-4-(4-chloro-1-methyl-1*H*-pyrazol-5-yl)-2-furancarboxamide (Compound B) | 60mg |
| Mannitol | 250 mg |
| Talc | 125 mg |
| Magnesium Stearate | 8mg |

### Example 2 - Capsule Composition

An oral dosage form for administering one of the compounds of the present invention is produced by filing a standard two piece hard gelatin capsule with the ingredients in the proportions shown in Table II, below.

Table II

| INGREDIENTS | AMOUNTS |
|---|---|
| N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide (the dimethyl sulfoxide solvate of Compound A) | 5mg |
| Mannitol | 55 mg |
| Talc | 16 mg |
| Magnesium Stearate | 4 mg |

### Example 3 - Capsule Composition

An oral dosage form for administering one of the compounds of the present invention is produced by filing a standard two piece hard gelatin capsule with the ingredients in the proportions shown in Table III, below.

Table III

| INGREDIENTS | AMOUNTS |
|---|---|
| *N*-{(1*S*)-2-amino-1-[(3,4-difluorophenyl)methyl]ethyl}-5-chloro-4-(4-chloro-1-methyl-1*H*-pyrazol-5-yl)-2-furancarboxamide (Compound B) | 60mg |
| Mannitol | 250mg |

(continued)

| INGREDIENTS | AMOUNTS |
|---|---|
| Talc | 125mg |
| Magnesium Stearate | 8mg |

Example 4 - Tablet Composition

The sucrose, microcrystalline cellulose and the compounds of the invented combination, as shown in Table IV below, are mixed and granulated in the proportions shown with a 10% gelatin solution. The wet granules are screened, dried, mixed with the starch, talc and stearic acid, then screened and compressed into a tablet.

Table IV

| INGREDIENTS | AMOUNTS |
|---|---|
| N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide (the dimethyl sulfoxide solvate of Compound A) | 5mg |
| *N*-{(1*S*)-2-amino-1-[(3,4-difluorophenyl)methyl]ethyl}-5-chloro-4-(4-chloro-1-methyl-1*H*-pyrazol-5-yl)-2-furancarboxamide (Compound B) | 60mg |
| Microcrystalline cellulose | 300mg |
| sucrose | 10mg |
| starch | 40mg |
| talc | 20mg |
| stearic acid | 5mg |

Example 5 - Tablet Composition

The sucrose, microcrystalline cellulose and one of the compounds of the invented combination, as shown in Table V below, are mixed and granulated in the proportions shown with a 10% gelatin solution. The wet granules are screened, dried, mixed with the starch, talc and stearic acid, then screened and compressed into a tablet.

Table V

| INGREDIENTS | AMOUNTS |
|---|---|
| N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide (the dimethyl sulfoxide solvate of Compound A) | 5mg |
| Microcrystalline cellulose | 30mg |
| sucrose | 4mg |
| starch | 2mg |
| talc | 1mg |
| stearic acid | 0.5mg |

Example 6 - Tablet Composition

The sucrose, microcrystalline cellulose and one of the compounds of the invented combination, as shown in Table VI below, are mixed and granulated in the proportions shown with a 10% gelatin solution. The wet granules are screened, dried, mixed with the starch, talc and stearic acid, then screened and compressed into a tablet.

Table VI

| INGREDIENTS | AMOUNTS |
|---|---|
| *N*-{(1*S*)-2-amino-1-[(3,4-difluorophenyl)methyl]ethyl}-5-chloro-4-(4-chloro-1-methyl-1*H*-pyrazol-5-yl)-2-furancarboxamide (Compound B) | 60mg |
| Microcrystalline cellulose | 300mg |
| sucrose | 40mg |

(continued)

| INGREDIENTS | AMOUNTS |
|---|---|
| starch | 20mg |
| talc | 10mg |
| stearic acid | 5mg |

## Claims

1. A combination comprising:

   (i) a compound of Structure (I):

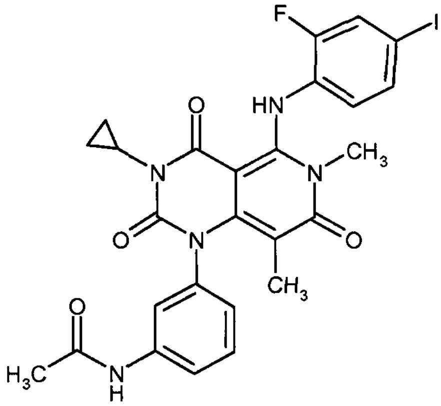

(I);

   or a pharmaceutically acceptable salt or solvate thereof; and
   (ii) a compound of Structure (II):

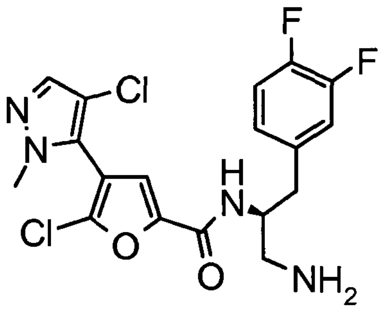

(II)

   or a pharmaceutically acceptable salt thereof.

2. A combination according to claim 1 where the compound of Structure (I) is in the form of a dimethyl sulfoxide solvate.

3. A combination kit comprising a combination according to claim 1 or claim 2 together with a pharmaceutically acceptable carrier or carriers.

4. Use of a combination according to claim 1 or claim 2 or of a combination kit according to claim 3 in the manufacture of a medicament or medicaments for the treatment of cancer or pre-cancerous syndromes.

5. A combination or combination kit as claimed in any one of claims 1 to 3 for use in the treatment of cancer or pre-cancerous syndromes.

6. A combination or combination kit for use in the treatment of cancer or pre-cancerous syndromes as claimed in claim

5, comprising a therapeutically effective amount of a combination of N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide solvate and N-{(1S)-2-amino-1-[(3,4-difluorophenyl)methyl]ethyl}-5-chloro-4-(4-chloro-1-methyl-1H-pyrazol-5-yl)-2-furancarboxamide or a pharmaceutically acceptable salt thereof,
wherein the combination is administered within a specified period, and
wherein the combination is administered for a duration of time.

**7.** A combination or combination kit for use according to claim 6 wherein the amount of N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide is selected from 0.25mg to 9mg, and that amount is administered once per day, and the amount of N-{(1S)-2-amino-1-[(3,4-difluorophenyl)methyl]ethyl}-5-chloro-4-(4-chloro-1-methyl-1H-pyrazol-5-yl)-2-furancarboxamide or a pharmaceutically acceptable salt thereof, is selected from 10mg to 300mg, and that amount is administered once per day.

**8.** A combination or combination kit for use according to claim 6 wherein N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide and N-{(1S)-2-amino-1-[(3,4-difluorophenyl)methyl]ethyl}-5-chloro-4-(4-chloro-1-methyl-1H-pyrazol-5-yl)-2-furancarboxamide or a pharmaceutically acceptable salt thereof, are administered within 12 hours of each other for from 1 to 3 consecutive days followed by administration of N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide for from 3 to 7 consecutive days, optionally followed by one or more cycles of repeat dosing.

**9.** A combination or combination kit for use according to claim 8 wherein N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide and N-{(1S)-2-amino-1-[(3,4-difluorophenyl)methyl]ethyl}-5-chloro-4-(4-chloro-1-methyl-1H-pyrazol-5-yl)-2-furancarboxamide or a pharmaceutically acceptable salt thereof are administered within 12 hours of each other for 2 consecutive days followed by administration of N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide for from 4 to 6 consecutive days, optionally followed by one or more cycles of repeat dosing.

**10.** A combination or combination kit for use according to claim 6 wherein N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide and N-{(1S)-2-amino-1-[(3,4-difluorophenyl)methyl]ethyl}-5-chloro-4-(4-chloro-1-methyl-1H-pyrazol-5-yl)-2-furancarboxamide or a pharmaceutically acceptable salt thereof are administered within 12 hours of each other for 2 days over a 7 day period, and during the other days of the 7 day period: either N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide is administered alone, optionally followed by one or more cycles of repeat dosing; or N-{(1S)-2-amino-1-[(3,4-difluorophenyl)methyl]ethyl}-5-chloro-4-(4-chloro-1-methyl-1H-pyrazol-5-yl)-2-furancarboxamide or a pharmaceutically acceptable salt thereof is administered alone, optionally followed by one or more cycles of repeat dosing.

**11.** A combination or combination kit for use according to claim 6 wherein N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide and N-{(1S)-2-amino-1-[(3,4-difluorophenyl)methyl]ethyl}-5-chloro-4-(4-chloro-1-methyl-1H-pyrazol-5-yl)-2-furancarboxamide or a pharmaceutically acceptable salt thereof are administered for at least 7 consecutive days.

**12.** A combination or combination kit for use according to claim 6 wherein N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide and N-{(1 S)-2-amino-1-[(3,4-difluorophenyl)methyl]ethyl}-5-chloro-4-(4-chloro-1-methyl-1H-pyrazol-5-yl)-2-furancarboxamide or a pharmaceutically acceptable salt thereof are administered within 12 hours of each other for 5 days over a 14 day period, and during the other days of the 14 day period: either N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide is administered alone, optionally followed by one or more cycles of repeat dosing; or N-{(1S)-2-amino-1-[(3,4-difluorophenyl)methyl]ethyl}-5-chloro-4-(4-chloro-1-methyl-1 H-pyrazol-5-yl)-2-furancarboxamide or a pharmaceutically acceptable salt thereof is administered alone, optionally followed by one or more cycles of repeat dosing.

**13.** A combination or combination kit for use according to claim 6 or claim 7 wherein the compound N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide is first administered in a loading dose for from 1 to 3 days followed by maintenance dose administration of the compound; and/or the compound N-{(1S)-2-amino-1-[(3,4-difluorophenyl)methyl]ethyl}-5-chloro-4-(4-chloro-1-methyl-1H-pyrazol-5-yl)-2-furancarboxamide or a pharmaceutically acceptable salt thereof is first administered in a loading dose for from 1 to 3 days followed by maintenance dose administration of the compound.

**14.** A use according to claim 4, or a combination or combination kit for use according to any one of claims 5 to 13, wherein the cancer is either wild type or mutant for Ras/Raf and either wild type or mutant for PI3K/PTEN.

**15.** A use according to claim 4, or a combination or combination kit for use according to any one of claims 5 to 13, wherein the cancer is selected from ovarian, breast, pancreatic and prostate.

**Patentansprüche**

**1.** Eine Kombination, umfassend:

(i) eine Verbindung der Struktur (I):

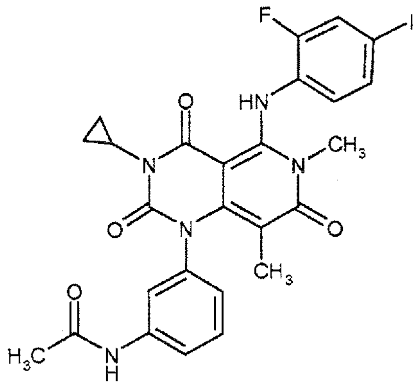

(I);

oder ein pharmazeutisch verträgliches Salz oder Solvat davon; und
(ii) eine Verbindung der Struktur (II):

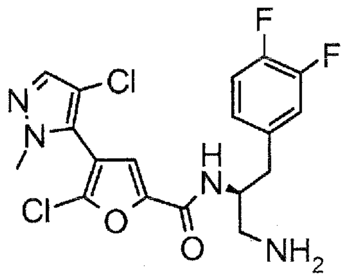

(II)

oder ein pharmazeutisch verträgliches Salz davon.

**2.** Eine Kombination nach Anspruch 1, wobei die Verbindung der Struktur (I) in Form eines Dimethylsulfoxidsolvats vorliegt.

**3.** Ein Kombinationskit, umfassend eine Kombination nach Anspruch 1 oder Anspruch 2 zusammen mit einem pharmazeutisch verträglichen Träger oder Trägern.

4. Verwendung einer Kombination nach Anspruch 1 oder Anspruch 2 oder eines Kombinationskits nach Anspruch 3 bei der Herstellung eines Medikaments oder von Medikamenten zur Behandlung von Krebs oder präkanzerösen Syndromen.

5. Eine Kombination oder ein Kombinationskit wie in einem der Ansprüche 1 bis 3 beansprucht zur Verwendung bei der Behandlung von Krebs oder präkanzerösen Syndromen.

6. Eine Kombination oder ein Kombinationskit zur Verwendung bei der Behandlung von Krebs oder präkanzerösen Syndromen wie in Anspruch 5 beansprucht, umfassend eine therapeutisch wirksame Menge einer Kombination von N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamiddimethylsulfoxidsolvat und N-{(1S)-2-Amino-1-[(3,4-difluorphenyl)methyl]ethyl}-5-chlor-4-(4-chlor-1-methyl-1H-pyrazol-5-yl)-2-furancarboxamid oder eines pharmazeutisch verträglichen Salzes davon, wobei die Kombination innerhalb eines bestimmten Zeitraums verabreicht wird und wobei die Kombination für eine Zeitdauer verabreicht wird.

7. Eine Kombination oder ein Kombinationskit zur Verwendung nach Anspruch 6, wobei die Menge von N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamiddimethylsulfoxid ausgewählt ist aus 0,25 mg bis 9 mg und diese Menge einmal pro Tag verabreicht wird und die Menge von N-{(1S)-2-Amino-1-[(3,4-difluorphenyl)methyl]ethyl}-5-chlor-4-(4-chlor-1-methyl-1H-pyrazol-5-yl)-2-furancarboxamid oder eines pharmazeutisch verträglichen Salzes davon ausgewählt ist aus 10 mg bis 300 mg und diese Menge einmal pro Tag verabreicht wird.

8. Eine Kombination oder ein Kombinationskit zur Verwendung nach Anspruch 6, wobei N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamiddimethylsulfoxid und N-{(1S)-2-Amino-1-[(3,4-difluorphenyl)methyl]ethyl}-5-chlor-4-(4-chlor-1-methyl-1H-pyrazol-5-yl)-2-furancarboxamid oder ein pharmazeutisch verträgliches Salz davon jeweils im Abstand von 12 Stunden für 1 bis 3 aufeinanderfolgende Tage verabreicht werden, gefolgt von einer Verabreichung von N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamiddimethylsulfoxid für 3 bis 7 aufeinanderfolgende Tage, gegebenenfalls gefolgt von einem oder mehreren Zyklen wiederholter Dosierung.

9. Eine Kombination oder ein Kombinationskit zur Verwendung nach Anspruch 8, wobei N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamiddimethylsulfoxid und N-{(1S)-2-Amino-1-[(3,4-difluorphenyl)methyl]ethyl}-5-chlor-4-(4-chlor-1-methyl-1H-pyrazol-5-yl)-2-furancarboxamid oder ein pharmazeutisch verträgliches Salz davon jeweils im Abstand von 12 Stunden für 2 aufeinanderfolgende Tage verabreicht werden, gefolgt von einer Verabreichung von N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamiddimethylsulfoxid für 4 bis 6 aufeinanderfolgende Tage, gegebenenfalls gefolgt von einem oder mehreren Zyklen wiederholter Dosierung.

10. Eine Kombination oder ein Kombinationskit zur Verwendung nach Anspruch 6, wobei N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamiddimethylsulfoxid und N-{(1S)-2-Amino-1-[(3,4-difluorphenyl)methyl]ethyl}-5-chlor-4-(4-chlor-1-methyl-1H-pyrazol-5-yl)-2-furancarboxamid oder ein pharmazeutisch verträgliches Salz davon jeweils im Abstand von 12 Stunden für 2 Tage über einen Zeitraum von 7 Tagen verabreicht werden und in den anderen Tagen des Zeitraums von 7 Tagen: entweder N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamiddimethylsulfoxid allein verabreicht wird, gegebenenfalls gefolgt von einem oder mehreren Zyklen wiederholter Dosierung; oder N-{(1S)-2-Amino-1-[(3,4-difluorphenyl)methyl]ethyl}-5-chlor-4-(4-chlor-1-methyl-1H-pyrazol-5-yl)-2-furancarboxamid oder ein pharmazeutisch verträgliches Salz davon alleine verabreicht wird, gegebenenfalls gefolgt von einem oder mehreren Zyklen wiederholter Dosierung.

11. Eine Kombination oder ein Kombinationskit zur Verwendung nach Anspruch 6, wobei N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamiddimethylsulfoxid und N-{(1S)-2-Amino-1-[(3,4-difluorphenyl)methyl]ethyl}-5-chlor-4-(4-chlor-1-methyl-1H-pyrazol-5-yl)-2-furancarboxamid oder ein pharmazeutisch verträgliches Salz davon für mindestens 7 aufeinanderfolgende Tage verabreicht werden.

12. Eine Kombination oder ein Kombinationskit zur Verwendung nach Anspruch 6, wobei N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamiddimethylsulfoxid und N-{(1S)-2-Amino-1-[(3,4-difluorphenyl)methyl]ethyl}-5-chlor-4-(4-chlor-1-methyl-1H-pyrazol-5-yl)-2-furancarboxamid oder ein pharmazeutisch verträgliches Salz davon jeweils im Abstand von 12 Stunden für 5 Tage über einen Zeitraum von 14 Tagen verabreicht werden und in den anderen Tagen des Zeitraums von 14 Tagen: entweder N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamiddimethyl-sulfoxid allein verabreicht wird, gegebenenfalls gefolgt von einem oder mehreren Zyklen wiederholter Dosierung; oder N-{(1S)-2-Amino-1-[(3,4-difluorphenyl)methyl]ethyl}-5-chlor-4-(4-chlor-1-methyl-1H-pyrazol-5-yl)-2-furancarboxamid oder ein pharmazeutisch verträgliches Salz davon allein verabreicht wird, gegebenenfalls gefolgt von einem oder mehreren Zyklen wiederholter Dosierung.

13. Eine Kombination oder ein Kombinationskit zur Verwendung nach Anspruch 6 oder Anspruch 7, wobei die Verbindung N-{3-[3-Cyclopropyl-5-(2-fluor-4-iod-phenylamino)-6,8-dimethyl-2,4,7-trioxo-3,4,6,7-tetrahydro-2H-pyrido[4,3-d]pyrimidin-1-yl]phenyl}acetamiddimethylsulfoxid zuerst in einer Belastungsdosis für 1 bis 3 Tage verabreicht wird, gefolgt von einer Verabreichung einer Erhaltungsdosis der Verbindung; und/oder die Verbindung N-{(1S)-2-Amino-1-[(3,4-difluorphenyl)methyl]ethyl}-5-chlor-4-(4-chlor-1-methyl-1H-pyrazol-5-yl)-2-furancarboxamid oder ein pharmazeutisch verträgliches Salz davon zuerst in einer Belastungsdosis für 1 bis 3 Tage verabreicht wird, gefolgt von einer Verabreichung einer Erhaltungsdosis der Verbindung.

14. Eine Verwendung nach Anspruch 4 oder eine Kombination oder ein Kombinationskit zur Verwendung nach einem der Ansprüche 5 bis 13, wobei der Krebs entweder vom Wildtyp oder eine Mutation für Ras/Raf ist und entweder vom Wildtyp oder eine Mutation für PI3K/PTEN ist.

15. Eine Verwendung nach Anspruch 4 oder eine Kombination oder ein Kombinationskit zur Verwendung nach einem der Ansprüche 5 bis 13, wobei der Krebs ausgewählt ist aus Eierstock, Brust, Pankreas und Prostata.

**Revendications**

1. Association comprenant :

(i) un composé de Structure (I) :

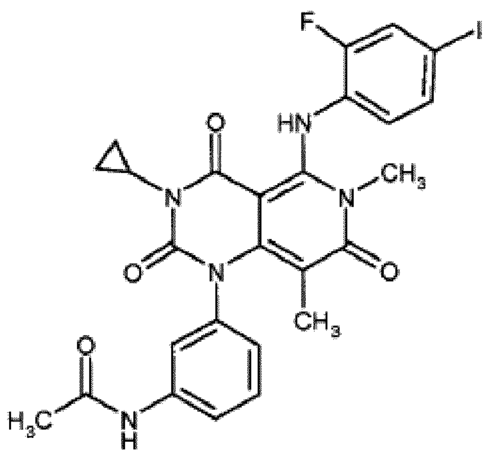

(I);

ou un de ses sels ou produits de solvatation pharmaceutiquement acceptables ; et
(ii) un composé de Structure (II) :

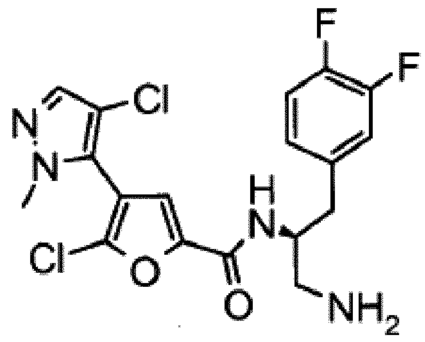

(II)

ou un de ses sels pharmaceutiquement acceptables.

2. Association suivant la revendication 1, dans laquelle le composé de Structure (I) est sous forme d'un produit de solvatation de diméthylsulfoxyde.

3. Kit d'association comprenant une association suivant la revendication 1 ou la revendication 2, conjointement avec un ou plusieurs supports pharmaceutiquement acceptables.

4. Utilisation d'une association suivant la revendication 1 ou la revendication 2 ou d'un kit d'association suivant la revendication 3 dans la production d'un ou plusieurs médicaments pour le traitement du cancer ou de syndromes précancéreux.

5. Association ou kit d'association suivant l'une quelconque des revendications 1 à 3, pour une utilisation dans le traitement du cancer ou de syndromes précancéreux.

6. Association ou kit d'association pour une utilisation dans le traitement du cancer ou de syndromes précancéreux suivant la revendication 5, comprenant une quantité thérapeutiquement efficace d'une association de produit de solvatation de diméthylsulfoxyde de N-{3-[3-cyclopropyl-5-(2-fluoro-4-iodo-phénylamino)-6,8-diméthyl-2,4,7-trioxo-3,4,6,7-tétrahydro-2H-pyrido[4,3-d]pyrimidine-1-yl]phényl}acétamide et de N-{(1S)-2-amino-1-[(3,4-difluoro-phényl)méthyl]éthyl}-5-chloro-4-(4-chloro-1-méthyl-1H-pyrazole-5-yl)-2-furanne-carboxamide, ou d'un de ses sels pharmaceutiquement acceptables,
l'association étant administrée en une période de temps spécifiée, et
l'association étant administrée pendant une certaine durée.

7. Association ou kit d'association pour une utilisation suivant la revendication 6, dans lequel la quantité de diméthylsulfoxyde-N-{3-[3-cyclopropyl-5-(2-fluoro-4-iodo-phénylamino)-6,8-diméthyl-2,4,7-trioxo-3,4,6,7-tétrahydro-2H-pyrido[4,3-d]pyrimidine-1-yl]phényl}acétamide est choisie entre 0,25 mg et 9 mg, et cette quantité est administrée une fois par jour, et la quantité de N-{(1S)-2-amino-1-[(3,4-difluorophényl)méthyl]éthyll-5-chloro-4-(4-chloro-1-méthyl-1H-pyrazole-5-yl)-2-furannecarboxamide ou d'un de ses sels pharmaceutiquement acceptables est choisie entre 10 mg et 300 mg et cette quantité est administrée une fois par jour.

8. Association ou kit d'association pour une utilisation suivant la revendication 6, dans lequel le diméthylsulfoxyde-N-{3-[3-cyclopropyl-5-(2-fluoro-4-iodo-phénylamino)-6,8-diméthyl-2,4,7-trioxo-3,4,6,7-tétrahydro-2H-pyrido[4,3-d]pyrimidine-1-yl]phényl}acétamide et le N-{(1S)-2-amino-1-[(3,4-difluorophényl)méthyl]éthyl}-5-chloro-4-(4-chloro-1-méthyl-1H-pyrazole-5-yl)-2-furannecarboxamide ou un de ses sels pharmaceutiquement acceptables sont administrés dans un intervalle de temps de 12 heures l'un par rapport à l'autre pendant 1 à 3 jours consécutifs, avec ensuite l'administration de diméthylsulfoxyde-N-{3-[3-cyclopropyl-5-(2-fluoro-4-iodo-phénylamino)-6,8-diméthyl-2,4,7-trioxo-3,4,6,7-tétrahydro-2H-pyrido[4,3-d]pyrimidine-1-yl]phényl}-acétamide pendant 3 à 7 jours consécutifs, avec ensuite facultativement un ou plusieurs cycles d'administration répétée.

9. Association ou kit d'association pour une utilisation suivant la revendication 8, dans lequel le diméthylsulfoxyde-N-{3-[3-cyclopropyl-5-(2-fluoro-4-iodo-phényl-amino)-6,8-diméthyl-2,4,7-trioxo-3,4,6,7-tétrahydro-2H-pyrido-[4,3-d]pyrimidine-1-yl]phényl}acétamide et le N-{(1S)-2-amino-1-[(3,4-difluorophényl)méthyl]éthyl}-5-chloro-4-(4-chloro-1-méthyl-1H-pyrazole-5-yl)-2-furannecarboxamide ou un de ses sels pharmaceutiquement acceptables sont administrés dans un intervalle de temps de 12 heures l'un par rapport à l'autre pendant 2 jours consécutifs, avec ensuite

l'administration de diméthylsulfoxyde-N-{3-[3-cyclopropyl-5-(2-fluoro-4-iodophénylamino)-6,8-diméthyl-2,4,7-trioxo-3,4,6,7-tétrahydro-2H-pyrido[4,3-d]pyrimidine-1-yl]phényl}-acétamide pendant 4 à 6 jours consécutifs, avec ensuite facultativement 1 ou plusieurs cycles d'administration répétée.

**10.** Association ou kit d'association pour une utilisation suivant la revendication 6, dans lequel le diméthylsulfoxyde-N-{3-[3-cyclopropyl-5-(2-fluoro-4-iodo-phényl-amino)-6,8-diméthyl-2,4,7-trioxo-3,4,6,7-tétrahydro-2H-pyrido[4,3-d]pyrimidine-1-yl]phényl}acétamide et le N-{(1S)-2-amino-1-[(3,4-difluorophényl)méthyl]éthyl}-5-chloro-4-(4-chloro-1-méthyl-1H-pyrazole-5-yl)-2-furannecarboxamide ou un de ses sels pharmaceutiquement acceptables sont administrés dans un intervalle de temps de 12 heures l'un par rapport à l'autre pendant 2 jours au cours d'une période de temps de 7 jours et, pendant les autres jours de la période de temps de 7 jours : soit le diméthylsulfoxyde-N-{3-[3-cyclopropyl-5-(2-fluoro-4-iodo-phénylamino)-6,8-diméthyl-2,4,7-trioxo-3,4,6,7-tétrahydro-2H-pyrido[4,3-d]pyrimidine-1-yl]phényl}-acétamide est administré seul, avec ensuite facultativement un ou plusieurs cycles d'administration répétée ; soit le N-{(1S)-2-amino-1-[(3,4-difluorophényl)méthyl]éthyl}-5-chloro-4-(4-chloro-1-méthyl-1H-pyrazole-5-yl)-2-furanne-carboxamide ou un de sels pharmaceutiquement acceptables est administré seul, avec ensuite éventuellement un ou plusieurs cycles d'administration répétée.

**11.** Association ou kit d'association pour une utilisation suivant la revendication 6, dans lequel le diméthylsulfoxyde-N-{3-[3-cyclopropyl-5-(2-fluoro-4-iodo-phényl-amino)-6,8-diméthyl-2,4,7-trioxo-3,4,6,7-tétrahydro-2H-pyrido[4,3-d]pyrimidine-1-yl]phényl}acétamide et le N-{(1S)-2-amino-1-[(3,4-difluorophényl)méthyl]éthyl}-5-chloro-4-(4-chloro-1-méthyl-1H-pyrazole-5-yl)-2-furannecarboxamide ou un de ses sels pharmaceutiquement acceptables sont administrés pendant au moins 7 jours consécutifs.

**12.** Association ou kit d'association pour une utilisation suivant la revendication 6, dans lequel le diméthylsulfoxyde-N-{3-[3-cyclopropyl-5-(2-fluoro-4-iodo-phényl-amino)-6,8-diméthyl-2,4,7-trioxo-3,4,6,7-tétrahydro-2H-pyrido[4,3-d]pyrimidine-1-yl]phényl}acétamide et le N-{(1S)-2-amino-1-[(3,4-difluorophényl)méthyl]éthyl}-5-chloro-4-(4-chloro-1-méthyl-1H-pyrazole-5-yl)-2-furannecarboxamide ou un de ses sels pharmaceutiquement acceptables sont administrés dans un intervalle de temps de 12 heures l'un par rapport à l'autre pendant 5 jours au cours d'une période de temps de 14 jours et, pendant les autres jours de la période de temps de 14 jours : soit le diméthylsulfoxyde-N-{3-[3-cyclopropyl-5-(2-fluoro-4-iodo-phénylamino)-6,8-diméthyl-2,4,7-trioxo-3,4,6,7-tétrahydro-2H-pyrido[4,3-d]pyrimidine-1-yl]phényl}acétamide est administré seul, avec ensuite facultativement un ou plusieurs cycles d'administration répétée ; soit le N-{(1S)-2-amino-1-[(3,4-difluorophényl)-méthyl]éthyl}-5-chloro-4-(4-chloro-1-méthyl-1H-pyrazole-5-yl)-2-furanne-carboxamide ou un de ses sels pharmaceutiquement acceptables est administré seul, avec ensuite facultativement un ou plusieurs cycles d'administration répétée.

**13.** Association ou kit d'association pour une utilisation suivant la revendication 6 ou la revendication 7, dans lequel le composé consistant en diméthylsulfoxyde-N-{3-[3-cyclopropyl-5-(2-fluoro-4-iodo-phénylamino)-6,8-diméthyl-2,4,7-trioxo-3,4,6,7-tétrahydro-2H-pyrido[4,3-d]-pyrimidine-1-yl]phényl}acétamide est administré tout d'abord à une dose d'attaque pendant 1 à 3 jours avec ensuite l'administration d'une dose d'entretien du composé ; et/ou le composé consistant en N-{(1S)-2-amino-1-[(3,4-difluorophényl)méthyl]éthyl}-5-chloro-4-(4-chloro-1-méthyl-1H-pyrazole-5-yl)-2-furannecarboxamide ou un de ses sels pharmaceutiquement acceptables est administré en premier à une dose d'attaque pendant 1 à 3 jours avec ensuite l'administration d'une dose d'entretien du composé.

**14.** Utilisation suivant la revendication 4, ou association ou kit d'association pour une utilisation suivant l'une quelconque des revendications 5 à 13, le cancer étant soit de type sauvage soit mutant pour Ras/Raf et soit de type sauvage soit mutant pour PI3K/PTEN.

**15.** Utilisation suivant la revendication 4, ou association ou kit d'association pour une utilisation suivant l'une quelconque des revendications 5 à 13, le cancer étant choisi entre les cancers des ovaires, du sein, du pancréas et de la prostate.

# Figure 1

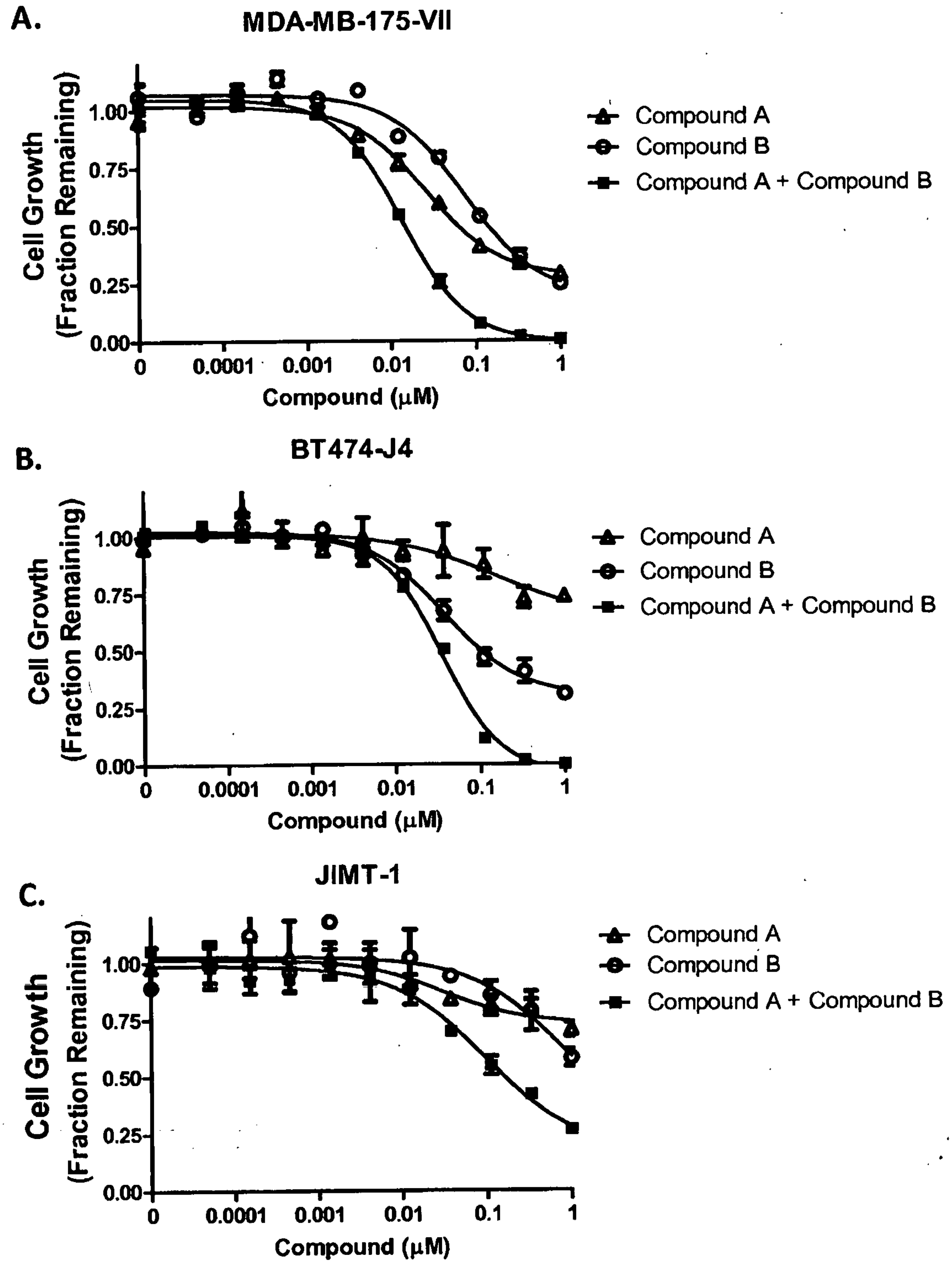
A.
MDA-MB-175-VII
Cell Growth (Fraction Remaining)
1.00
0.75
0.50
0.25
0.00
0
0.0001
0.001
0.01
0.1
1
Compound (μM)
Compound A
Compound B
Compound A + Compound B
B.
BT474-J4
Cell Growth (Fraction Remaining)
1.00
0.75
0.50
0.25
0.00
0
0.0001
0.001
0.01
0.1
1
Compound (μM)
Compound A
Compound B
Compound A + Compound B
C.
JIMT-1
Cell Growth (Fraction Remaining)
1.00
0.75
0.50
0.25
0.00
0
0.0001
0.001
0.01
0.1
1
Compound (μM)
Compound A
Compound B
Compound A + Compound B

# Figure 2

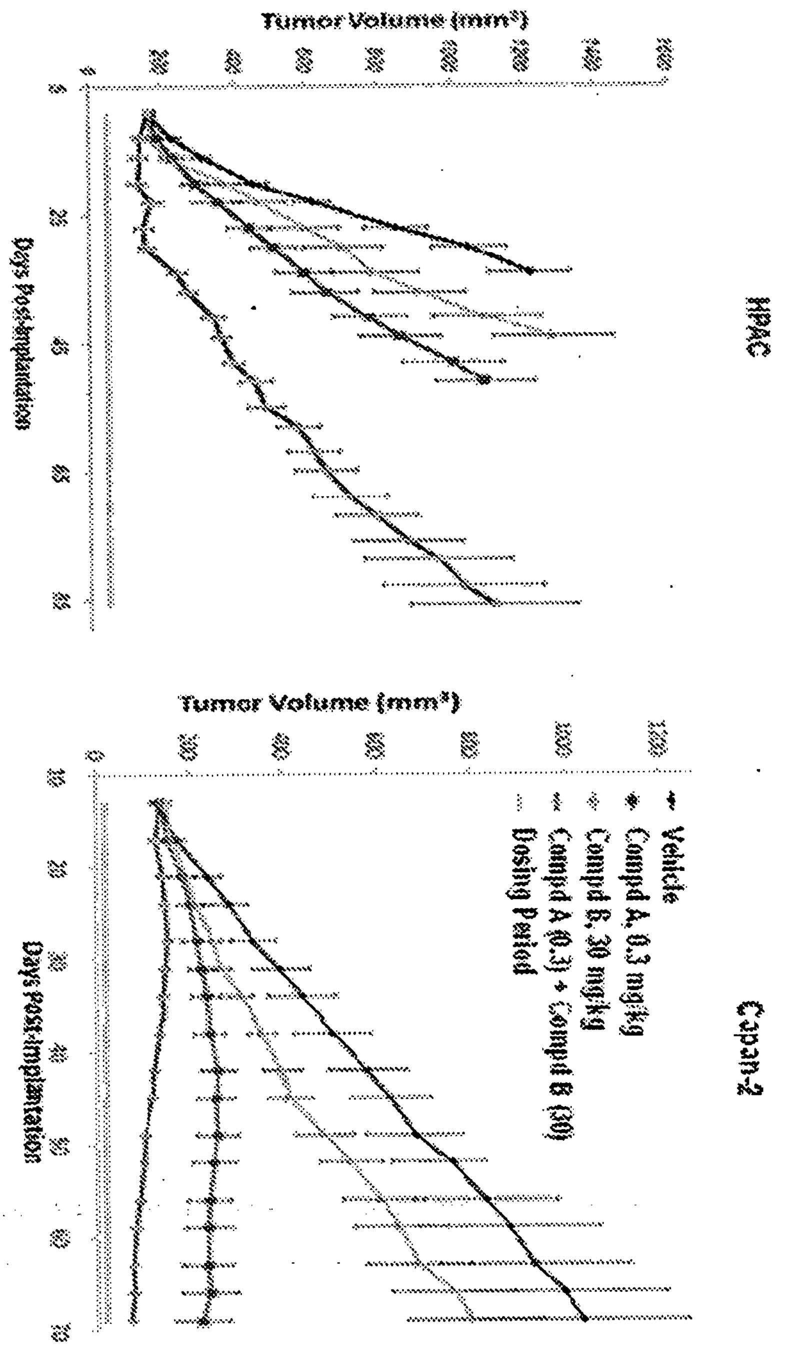
Tumor Volume (mm³)
Days Post-implantation
HPAC
Tumor Volume (mm³)
Days Post-implantation
Capan-2
Vehicle
Compd A, 0.3 mg/kg
Compd B, 30 mg/kg
Compd A (0.3) + Compd B (30)
Dosing Period

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005011082 W
- WO 2005121142 A
- US 20060014768 A
- US 2008053269 W
- WO 2008098104 A


### Non-patent literature cited in the description


- **CREWS ; ERIKSON.** *Cell,* 1993, vol. 74, 215-17
- **VIVANCO ; SAWYERS.** *Nat. Rev. Cancer,* 2002, vol. 2, 489-501
- **SAMUELS ; ERICSON.** *Curr. Opp in Oncology,* 2006, vol. 18, 77-82
- **HANAHAN ; WEINBERG.** *Cell,* 2000, vol. 100, 57-70
- **VIVANCO ; SAWYERS.** *Nat. Rev. Cancer.,* 2002, vol. 2, 489-501
- **CARPTEN et al.** *Nature,* 2007, vol. 448, 439-44
- **ALLEN, LF et al.** *Semin. Oncol.,* 2003, vol. 30 (5), 105-16
- **DAVIES, H. et al.** *Nature,* 2002, vol. 417, 949-54
- *The Journal of Biological Chemistry,* 2001, vol. 276 (4), 2686-2692
- **CHOU ; TALALAY.** *Advances in Enzyme Regulation,* 1984, vol. 22, 37-55
- **BERENBAUM, MC.** *Cancer Research,* 1981, vol. 35, 269-335
- **BORISY et al.** *FDA 21 CFR,* 2003, vol. 300, 50
- **BAMFORD S. et al.** *Br. J. Cancer.,* 2004, vol. 91, 355-58
- **RABBEE ; SPEED.** *Bioinformatics,* 2006, vol. 22, 7-12
- **CHOU TC ; TALALAY P.** Quantitative analysis of dose-effect relationships: the combined effects of multiple drugs or enzyme inhibitors. *Adv Enzyme Regul,* 1984, vol. 22, 27-55
- **PETERSON JJ ; NOVICK SJ.** Nonlinear blending: a useful general concept for the assessment of combination drug synergy. *J Recept Signal Transduct Res,* 2007, vol. 27 (2-3), 125-46
- **PETERSON J.** A Review of Synergy Concepts of Nonlinear Blending and Dose-Reduction Profiles. *Frontiers of Bioscience,* 2010, vol. S2, 483-503